# FASCICULE DE BREVET EUROPEEN

(11) **EP 1 673 155 B1**
(45) Date de publication et mention de la délivrance du brevet: **22.08.2012**
(21) Numéro de dépôt: 04817226.6
(22) Date de dépôt: 13.10.2004
(51) Int. Cl.: B01D 1/00

(54) **PROCEDE DE DISSOCIATION DE LA MOLECULE D'HEMOGLOBINE EXTRACELLULAIRE D'ARENICOLA MARINA, CARACTERISATION DES CHAINES PROTEIQUES CONSTITUANT LADITE MOLECULE ET DES SEQUENCES NUCLEOTIDIQUES CODANT POUR LESDITES CHAINES PROTEIQUES**
VERFAHREN ZUR DISSOZIATION DES EXTRAZELLULAREN HÄMOGLOBINMOLEKÜLS DES WATTWURMS UND DIE CHARAKTERISIERUNG DER DAS MOLEKÜL BILDENDEN PROTEINKETTEN UND DER NUKLEOTID-SEQUENZKODIERUNG FÜR DIE PROTEINKETTEN
METHOD FOR THE DISSOCIATION OF THE EXTRACELLULAR HAEMOGLOBIN MOLECULE OF ARENICOLA MARINA AND THE CHARACTERISATION OF THE PROTEIN CHAINS FORMING THE MOLECULE AND THE NUCLEOTIDE SEQUENCES CODING FOR SAID PROTEIN CHAINS

(30) Priorité: 14.10.2003 FR 0311992
(43) Date de publication de la demande: 28.06.2006
(73) Titulaire: Centre National de la Recherche Scientifique (CNRS), 75794 Paris Cédex 16 (FR); UNIVERSITE PIERRE ET MARIE CURIE, 75252 Paris Cédex 05 (FR)
(72) Inventeur: ZAL, Franck, F-29600 Morlaix-Ploujean (FR); CHABASSE, Christine, F-77176 Nandy (FR); ROUSSELOT, Morgane, F-29350 Mo¬lan s/Mer (FR); BAILLY, Xavier, F-27180 Caugé (FR)
(74) Mandataire: Cabinet Plasseraud
(86) Numéro de dépôt international: PCT/FR2004/002602
(87) Numéro de publication internationale: WO 2005/037392

(56) Documents cités:
- WO-A-01/92320
- KREBS A. ET AL: "molecular shape, dissociation, and oxygen binding of the dodecamer subunit of lumbricus terrestris hemoglobin" JOURNAL OF BIOLOGICAL CHEMISTRY, vol. 271, no. 31, 1996, pages 18695-18704, XP002272714
- PAWAN K. ET AL: "the role of the dodecamer subunit in the dissociation and reassembly of the hexagonal bilayer structure of lumbricus terrestris hemoglobin" JOURNAL OF BIOLOGICAL CHEMISTRY, vol. 271, no. 15, 1996, pages 8754-8762, XP002272715
- FUSHITANI K. ET AL.: "the extracellular hemoglobin of the earthworm lumbricus terrestris" JOURNAL OF BIOLOGICAL CHEMISTRY, vol. 266, no. 16, 1991, pages 10275-10281, XP002272716
- SGOUROS J. ET AL: "Invertebrate oxygen carriers" 1986, SPRINGER VERLAG , BERLIN-HEIDELBERG , XP009026675 le document en entier
- ZAL ET AL: "Quaternary structure of the extracellular hemoglobin of the lugworm Arenicola marina. A multi-angle-laser-light-scattering and electrospray-ionization-mass-spectrometry analysis" CHEMABS, 1997, XP002165190
- PIONETTI J.M. ET AL.: "molecular architecture of annelid erythrocruorins" EUR. J. BIOCHEM, vol. 105, 1980, pages 131-138, XP009026718

### LISTE DE SEQUENCES

<110> CENTRE NATIONAL DE LA RECHERCHE SCIENTIFIQUE
<120> PROCÉDÉ DE DISSOCIATION DE LA MOLÉCULE D'HÉMOGLOBINE EXTRACELLULAIRE D'ARENICOLA MARINA, CARACTÉRISATION DES CHAÎNES PROTÉIQUES CONSTITUANT LADITE MOLÉCULE ET DES SÉQUENCES NUCLÉOTIDIQUES CODANT POUR LESDITES CHAÎNES PROTÉIQUES
<130> WOB CNR GLOB
<150> FR 03/11992
   <151> 2003-10-14
<160> 31
<170> PatentIn version 3.1
<210> 1
   <211> 474
   <212> ADN
   <213> Arenicola marina
<220>
   <221> CDS
   <222> (1)..(474)
   <223>
<400> 1
   atg aag tcc ttg gtg gtt ctg ttc gcc ctg gtg gcc atg gtg gct gca       48
   Met Lys Ser Leu Val Val Leu Phe Ala Leu Val Ala Met Val Ala Ala
   1               5                   10                  15
   gag tgc ggc ccc atg cag cgc ctc ctg gtc aag acc cag tgg aac aag       96
   Glu Cys Gly Pro Met Gln Arg Leu Leu Val Lys Thr Gln Trp Asn Lys
               20                  25                  30
   gtg tac ggc acc agc aag gtc agg gac gag gcc gga cac gtc ctc tgg      144
   Val Tyr Gly Thr Ser Lys Val Arg Asp Glu Ala Gly His Val Leu Trp
           35                  40                  45
   aag gct att ttc gcc cag gat ccc gag acc cgg gct ctc ttc aag aga      192
   Lys Ala Ile Phe Ala Gln Asp Pro Glu Thr Arg Ala Leu Phe Lys Arg
       50                  55                  60
   gtc aac ggt gac gac atc tac tct ccc gag ttc atg gct cac agc gcc      240
   Val Asn Gly Asp Asp Ile Tyr Ser Pro Glu Phe Met Ala His Ser Ala
   65                  70                  75                  80
   cgt gtc ttg ggt ggc ctt gac att gcc atc tcc ctc ctc gac aac cag      288
   Arg Val Leu Gly Gly Leu Asp Ile Ala Ile Ser Leu Leu Asp Asn Gln
                   85                  90                  95
   gct gac ctt gac gtc gcc ctg gct cac ctt cac gtg cag cac gta gaa      336
   Ala Asp Leu Asp Val Ala Leu Ala His Leu His Val Gln His Val Glu
               100                 105                 110
   agg cac atc cca acc cgc tac ttc gat ctg ttc aag aac gcc ctg atg      384
   Arg His Ile Pro Thr Arg Tyr Phe Asp Leu Phe Lys Asn Ala Leu Met
           115                 120                 125
   gag tat gcc ccc agc gcc ctg gga cgc tgc ttc gat aag acc gcc tgg      432
   Glu Tyr Ala Pro Ser Ala Leu Gly Arg Cys Phe Asp Lys Thr Ala Trp
       130                 135                 140
   agc tcg tgc ttt gac gtc atc gcc aac ggc atc aag gaa tag              474
   Ser Ser Cys Phe Asp Val Ile Ala Asn Gly Ile Lys Glu
   145                 150                 155
<210> 2
   <211> 157
   <212> PRT
   <213> Arenicola marina
<400> 2
   Met Lys Ser Leu Val Val Leu Phe Ala Leu Val Ala Met Val Ala Ala
   1               5                   10                  15
   Glu Cys Gly Pro Met Gln Arg Leu Leu Val Lys Thr Gln Trp Asn Lys
               20                  25                  30
   Val Tyr Gly Thr Ser Lys Val Arg Asp Glu Ala Gly His Val Leu Trp
           35                  40                  45
   Lys Ala Ile Phe Ala Gln Asp Pro Glu Thr Arg Ala Leu Phe Lys Arg
       50                  55                  60
   Val Asn Gly Asp Asp Ile Tyr Ser Pro Glu Phe Met Ala His Ser Ala
   65                  70                  75                  80
   Arg Val Leu Gly Gly Leu Asp Ile Ala Ile Ser Leu Leu Asp Asn Gln
                   85                  90                  95
   Ala Asp Leu Asp Val Ala Leu Ala His Leu His Val Gln His Val Glu
               100                 105                 110
   Arg His Ile Pro Thr Arg Tyr Phe Asp Leu Phe Lys Asn Ala Leu Met
           115                 120                 125
   Glu Tyr Ala Pro Ser Ala Leu Gly Arg Cys Phe Asp Lys Thr Ala Trp
       130                 135                 140
   Ser Ser Cys Phe Asp Val Ile Ala Asn Gly Ile Lys Glu
   145                 150                 155
<210> 3
   <211> 477
   <212> ADN
   <213> Arenicola marina
<220>
   <221> CDS
   <222> (1)..(477)
   <223>
<400> 3
   atg aag ttc ttg gtg gtt ctg ttc gcc ctg gtg gcc atg gtc gct gct       48
   Met Lys Phe Leu Val Val Leu Phe Ala Leu Val Ala Met Val Ala Ala
   1               5                   10                  15
   gat tgt ggc ccc atg cag cgc ctc ctg gtc aag gcc cag tgg aac aag        96
   Asp Cys Gly Pro Met Gln Arg Leu Leu Val Lys Ala Gln Trp Asn Lys
               20                  25                  30
   gtg tac ggc acc agc aag gtc agg gac gac gcc gga cac gtc ctc tgg       144
   Val Tyr Gly Thr Ser Lys Val Arg Asp Asp Ala Gly His Val Leu Trp
           35                  40                  45
   aag gct atc ttc aac cag gat ggt gag acc cgc gcc ctc ttc aac aga       192
   Lys Ala Ile Phe Asn Gln Asp Gly Glu Thr Arg Ala Leu Phe Asn Arg
       50                  55                  60
   gtg cac ggt gac gac atc tac tct ccc gag ttc atg gct cac agc gcc       240
   Val His Gly Asp Asp Ile Tyr Ser Pro Glu Phe Met Ala His Ser Ala
   65                  70                  75                  80
   cgt gtc ttg ggt ggc ctt gac att gcc atc tcc ctc ctc gac aac cag       288
   Arg Val Leu Gly Gly Leu Asp Ile Ala Ile Ser Leu Leu Asp Asn Gln
                   85                  90                  95
   gct gag ctt gac gct gtc ctg gct cac ctc aag gag cag cac att gag       336
   Ala Glu Leu Asp Ala Val Leu Ala His Leu Lys Glu Gln His Ile Glu
               100                 105                 110
   agg ggg atc cca gac cgt tac ttc gac ctg ttc aag aac gcc ctg atg       384
   Arg Gly Ile Pro Asp Arg Tyr Phe Asp Leu Phe Lys Asn Ala Leu Met
           115                 120                 125
   gag ttt gcc ccc agc gcc ttg gga cgc tgc ttc ata aag gac gct tgg       432
   Glu Phe Ala Pro Ser Ala Leu Gly Arg Cys Phe Ile Lys Asp Ala Trp
       130                 135                 140
   agc tca tgc ttt gac gtc att gcc aac ggc atc aag gga cag taa           477
   Ser Ser Cys Phe Asp Val Ile Ala Asn Gly Ile Lys Gly Gln
   145                 150                 155
<210> 4
   <211> 158
   <212> PRT
   <213> Arenicola marina
<400> 4
   Met Lys Phe Leu Val Val Leu Phe Ala Leu Val Ala Met Val Ala Ala
   1               5                   10                  15
   Asp Cys Gly Pro Met Gln Arg Leu Leu Val Lys Ala Gln Trp Asn Lys
               20                  25                  30
   Val Tyr Gly Thr Ser Lys Val Arg Asp Asp Ala Gly His Val Leu Trp
           35                  40                  45
   Lys Ala Ile Phe Asn Gln Asp Gly Glu Thr Arg Ala Leu Phe Asn Arg
       50                  55                  60
   Val His Gly Asp Asp Ile Tyr Ser Pro Glu Phe Met Ala His Ser Ala
   65                  70                  75                  80
   Arg Val Leu Gly Gly Leu Asp Ile Ala Ile Ser Leu Leu Asp Asn Gln
                   85                  90                  95
   Ala Glu Leu Asp Ala Val Leu Ala His Leu Lys Glu Gln His Ile Glu
               100                 105                 110
   Arg Gly Ile Pro Asp Arg Tyr Phe Asp Leu Phe Lys Asn Ala Leu Met
           115                 120                 125
   Glu Phe Ala Pro Ser Ala Leu Gly Arg Cys Phe Ile Lys Asp Ala Trp
       130                 135                 140
   Ser Ser Cys Phe Asp Val Ile Ala Asn Gly Ile Lys Gly Gln
   145                 150                 155
<210> 5
   <211> 474
   <212> ADN
   <213> Arenicola marina
<220>
   <221> CDS
   <222> (1)..(474)
   <223>
<400> 5
   atg aag gtc ctg atc gta ctg atg gcc tgc ttg gcc tac gtc gcc gcc      48
   Met Lys Val Leu Ile Val Leu Met Ala Cys Leu Ala Tyr Val Ala Ala
   1               5                   10                  15
   gac tgc gga cct ctg cag agg ctg aag gtg aag cat cag tgg gtg cag      96
   Asp Cys Gly Pro Leu Gln Arg Leu Lys Val Lys His Gln Trp Val Gln
               20                  25                  30
   gtg tac agc ggc cat ggt tac gag cgt gag gcg ttc ggc aga gag gtc      144
   Val Tyr Ser Gly His Gly Tyr Glu Arg Glu Ala Phe Gly Arg Glu Val
           35                  40                  45
   ttc ctc gag atg tac aac cag gca ccc aag gcc aag gac ctc ttc acc      192
   Phe Leu Glu Met Tyr Asn Gln Ala Pro Lys Ala Lys Asp Leu Phe Thr
       50                  55                  60
   agg gtc agg ggc gag aac gtc ttc tcc ccc gag ttc gga gcc cac atg      240
   Arg Val Arg Gly Glu Asn Val Phe Ser Pro Glu Phe Gly Ala His Met
   65                  70                  75                  80
   gtc cgt gtg ctc gga gga ctc gac atg tgc atc gct ctg ctg tcc gat      288
   Val Arg Val Leu Gly Gly Leu Asp Met Cys Ile Ala Leu Leu Ser Asp
                   85                  90                  95
   gac acc gtc ctc aac gcc cag ctt gct cac ctc agc acg cag cac aag      336
   Asp Thr Val Leu Asn Ala Gln Leu Ala His Leu Ser Thr Gln His Lys
               100                 105                 110
   gac cgt gga atc ccc aac gag tac ttc gat gtg atg aag gtc gcc ctc      384
   Asp Arg Gly Ile Pro Asn Glu Tyr Phe Asp Val Met Lys Val Ala Leu
           115                 120                 125
   atg aag gtc gtc ccc ggc cac gtt tca cac ttc gac ttc gat gcc tgg      432
   Met Lys Val Val Pro Gly His Val Ser His Phe Asp Phe Asp Ala Trp
       130                 135                 140
   tct gcc tgc tat gac gtc atc gcc aac ggc atc aag cac taa              474
   Ser Ala Cys Tyr Asp Val Ile Ala Asn Gly Ile Lys His
   145                 150                 155
<210> 6
   <211> 157
   <212> PRT
   <213> Arenicola marina
<400> 6
   Met Lys Val Leu Ile Val Leu Met Ala Cys Leu Ala Tyr Val Ala Ala
   1               5                   10                  15
   Asp Cys Gly Pro Leu Gln Arg Leu Lys Val Lys His Gln Trp Val Gln
               20                  25                  30
   Val Tyr Ser Gly His Gly Tyr Glu Arg Glu Ala Phe Gly Arg Glu Val
           35                  40                  45
   Phe Leu Glu Met Tyr Asn Gln Ala Pro Lys Ala Lys Asp Leu Phe Thr
       50                  55                  60
   Arg Val Arg Gly Glu Asn Val Phe Ser Pro Glu Phe Gly Ala His Met
   65                  70                  75                  80
   Val Arg Val Leu Gly Gly Leu Asp Met Cys Ile Ala Leu Leu Ser Asp
                   85                  90                  95
   Asp Thr Val Leu Asn Ala Gln Leu Ala His Leu Ser Thr Gln His Lys
               100                 105                 110
   Asp Arg Gly Ile Pro Asn Glu Tyr Phe Asp Val Met Lys Val Ala Leu
           115                 120                 125
   Met Lys Val Val Pro Gly His Val Ser His Phe Asp Phe Asp Ala Trp
       130                 135                 140
   Ser Ala Cys Tyr Asp Val Ile Ala Asn Gly Ile Lys His
   145                 150                 155
<210> 7
   <211> 498
   <212> ADN
   <213> Arenicola marina
<220>
   <221> CDS
   <222> (1)..(498)
   <223>
<400> 7
   atg ctt cgt ttc gta gca ctc ttg gct ctg gtc ggc ctg gcc gtc tgt      48
   Met Leu Arg Phe Val Ala Leu Leu Ala Leu Val Gly Leu Ala Val Cys
   1               5                   10                  15
   gac gac tgt tgt acc acc gag gac cgc aag gag gtc cag acg ctg tgg      96
   Asp Asp Cys Cys Thr Thr Glu Asp Arg Lys Glu Val Gln Thr Leu Trp
               20                  25                  30
   agt gag atc tgg agt gcc cag ttc act ggt cgc cgt gtc cag gtt gcc      144
   Ser Glu Ile Trp Ser Ala Gln Phe Thr Gly Arg Arg Val Gln Val Ala
           35                  40                  45
   cag gct gtg ttc gag gac ctc ttc cgc cgc gac ccc gag tcc aag aac      192
   Gln Ala Val Phe Glu Asp Leu Phe Arg Arg Asp Pro Glu Ser Lys Asn
       50                  55                  60
   ctg ttc aag cgc gtc aat gtt gac gac atg aac agc ccc gaa ttc cac      240
   Leu Phe Lys Arg Val Asn Val Asp Asp Met Asn Ser Pro Glu Phe His
   65                  70                  75                  80
   gct cac tgc atc cgt gtt gtc aac ggt ctt gac acc gtg atc ggt ctc      288
   Ala His Cys Ile Arg Val Val Asn Gly Leu Asp Thr Val Ile Gly Leu
                   85                  90                  95
   ctt gac gac ccc gac acc ctg aag tcc cag ctc gag cac ttg gcc cag      336
   Leu Asp Asp Pro Asp Thr Leu Lys Ser Gln Leu Glu His Leu Ala Gln
               100                 105                 110
   cag cac aag gag cgt gat ggc atc cac aag acc cac ttc gac gag atg      384
   Gln His Lys Glu Arg Asp Gly Ile His Lys Thr His Phe Asp Glu Met
           115                 120                 125
   tcc cac gcc ttc ggc gcc gtc atg ccc cag gtc agc agc tgc ttc aac      432
   Ser His Ala Phe Gly Ala Val Met Pro Gln Val Ser Ser Cys Phe Asn
       130                 135                 140
   ccc gat gcc tgg aac cgt tgc ttc ggc tcc atc gct acc aag att gct      480
   Pro Asp Ala Trp Asn Arg Cys Phe Gly Ser Ile Ala Thr Lys Ile Ala
   145                 150                 155                 160
   tcc ctc ctc gag gat taa                                              498
   Ser Leu Leu Glu Asp
                   165
<210> 8
   <211> 165
   <212> PRT
   <213> Arenicola marina
<400> 8
   Met Leu Arg Phe Val Ala Leu Leu Ala Leu Val Gly Leu Ala Val Cys
   1               5                   10                  15
   Asp Asp Cys Cys Thr Thr Glu Asp Arg Lys Glu Val Gln Thr Leu Trp
               20                  25                  30
   Ser Glu Ile Trp Ser Ala Gln Phe Thr Gly Arg Arg Val Gln Val Ala
           35                  40                  45
   Gln Ala Val Phe Glu Asp Leu Phe Arg Arg Asp Pro Glu Ser Lys Asn
       50                  55                  60
   Leu Phe Lys Arg Val Asn Val Asp Asp Met Asn Ser Pro Glu Phe His
   65                  70                  75                  80
   Ala His Cys Ile Arg Val Val Asn Gly Leu Asp Thr Val Ile Gly Leu
                   85                  90                  95
   Leu Asp Asp Pro Asp Thr Leu Lys Ser Gln Leu Glu His Leu Ala Gln
               100                 105                 110
   Gln His Lys Glu Arg Asp Gly Ile His Lys Thr His Phe Asp Glu Met
           115                 120                 125
   Ser His Ala Phe Gly Ala Val Met Pro Gln Val Ser Ser Cys Phe Asn
       130                 135                 140
   Pro Asp Ala Trp Asn Arg Cys Phe Gly Ser Ile Ala Thr Lys Ile Ala
   145                 150                 155                 160
   Ser Leu Leu Glu Asp
                   165
<210> 9
   <211> 498
   <212> ADN
   <213> Arenicola marina
<220>
   <221> CDS
   <222> (1)..(498)
   <223>
<400> 9
   atg atg tcc gtc gtg ttc ctc ctc ggc ctt gtg gcc tac gcc tcc gcc        48
   Met Met Ser Val Val Phe Leu Leu Gly Leu Val Ala Tyr Ala Ser Ala
   1               5                   10                  15
   tcc agc tgc tgc tcc tat gga gac cag cag aag gtc aag gcc cag tgg        96
   Ser Ser Cys Cys Ser Tyr Gly Asp Gln Gln Lys Val Lys Ala Gln Trp
               20                  25                  30
   aac agc ctc tgg aac acc cct gac tcc tcc aca tcc aag atc atc ttc       144
   Asn Ser Leu Trp Asn Thr Pro Asp Ser Ser Thr Ser Lys Ile Ile Phe
           35                  40                  45
   gga aag gaa gtc ttc gca cgc ttc ttc gag gtt gac ccc gag agc aag       192
   Gly Lys Glu Val Phe Ala Arg Phe Phe Glu Val Asp Pro Glu Ser Lys
       50                  55                  60
   agc ctg ttc ggt cgc gtc aag gtt gaa gac ccc gac agc ccc gag ttc       240
   Ser Leu Phe Gly Arg Val Lys Val Glu Asp Pro Asp Ser Pro Glu Phe
   65                  70                  75                  80
   gcc gga cac gtg atc cgt gtt ttg acc ggt ctg gat ttg atc atc aac       288
   Ala Gly His Val Ile Arg Val Leu Thr Gly Leu Asp Leu Ile Ile Asn
                   85                  90                  95
   ttg atg ggt gac gat gcc atg gat gcc gag ctg gcc cac ctt aac acc       336
   Leu Met Gly Asp Asp Ala Met Asp Ala Glu Leu Ala His Leu Asn Thr
               100                 105                 110
   cag cat ttg gcc aga gag gga atc acc gga acc cac ttc acc gag atg       384
   Gln His Leu Ala Arg Glu Gly Ile Thr Gly Thr His Phe Thr Glu Met
           115                 120                 125
   ttc aag gtc ctg gat gga tcc ctc cgc cag gtt ctc gag gag tac gat       432
   Phe Lys Val Leu Asp Gly Ser Leu Arg Gln Val Leu Glu Glu Tyr Asp
       130                 135                 140
   tcc ctg tcc tgg agg tac tgc ttc cgt ggt ctg ggc gcc gcc ctc agg       480
   Ser Leu Ser Trp Arg Tyr Cys Phe Arg Gly Leu Gly Ala Ala Leu Arg
   145                 150                 155                 160
   gat ggt ctc ccc gca taa                                               498
   Asp Gly Leu Pro Ala
                   165
<210> 10
   <211> 165
   <212> PRT
   <213> Arenicola marina
<400> 10
   Met Met Ser Val Val Phe Leu Leu Gly Leu Val Ala Tyr Ala Ser Ala
   1               5                   10                  15
   Ser Ser Cys Cys Ser Tyr Gly Asp Gln Gln Lys Val Lys Ala Gln Trp
               20                  25                  30
   Asn Ser Leu Trp Asn Thr Pro Asp Ser Ser Thr Ser Lys Ile Ile Phe
           35                  40                  45
   Gly Lys Glu Val Phe Ala Arg Phe Phe Glu Val Asp Pro Glu Ser Lys
       50                  55                  60
   Ser Leu Phe Gly Arg Val Lys Val Glu Asp Pro Asp Ser Pro Glu Phe
   65                  70                  75                  80
   Ala Gly His Val Ile Arg Val Leu Thr Gly Leu Asp Leu Ile Ile Asn
                   85                  90                  95
   Leu Met Gly Asp Asp Ala Met Asp Ala Glu Leu Ala His Leu Asn Thr
               100                 105                 110
   Gln His Leu Ala Arg Glu Gly Ile Thr Gly Thr His Phe Thr Glu Met
           115                 120                 125
   Phe Lys Val Leu Asp Gly Ser Leu Arg Gln Val Leu Glu Glu Tyr Asp
       130                 135                 140
   Ser Leu Ser Trp Arg Tyr Cys Phe Arg Gly Leu Gly Ala Ala Leu Arg
   145                 150                 155                 160
   Asp Gly Leu Pro Ala
                   165
<210> 11
   <211> 771
   <212> ADN
   <213> Arenicola marina
<220>
   <221> CDS
   <222> (1)..(771)
   <223>
<400> 11
   atg aag agc tac gtg ctc gtg tgc tgc ctc gtg gtg ggg gcc gtg gcc      48
   Met Lys Ser Tyr Val Leu Val Cys Cys Leu Val Val Gly Ala Val Ala
   1               5                   10                  15
   tac ccc cac gag gtg atg cac cat gcc gtt ggc gca aac cga atg tgc      96
   Tyr Pro His Glu Val Met His His Ala Val Gly Ala Asn Arg Met Cys
               20                  25                  30
   aag tgt gat gcc ccg gca ggg aac gcc gaa acc tcc gcc gac aga gag      144
   Lys Cys Asp Ala Pro Ala Gly Asn Ala Glu Thr Ser Ala Asp Arg Glu
           35                  40                  45
   cag agt cac act ctc gat gag ttg acc cat cag ttg cac atg ctg cag      192
   Gln Ser His Thr Leu Asp Glu Leu Thr His Gln Leu His Met Leu Gln
       50                  55                  60
   caa gcc tac gac acc ggc atg ggt cgt gtc gat gac gtg atg gag gac      240
   Gln Ala Tyr Asp Thr Gly Met Gly Arg Val Asp Asp Val Met Glu Asp
   65                  70                  75                  80
   atg gac gac ctg tcc cac agg atc gcc gac cac gag aag gaa cac tgt      288
   Met Asp Asp Leu Ser His Arg Ile Ala Asp His Glu Lys Glu His Cys
                   85                  90                  95
   aag aag tat aga gag ttc cag tgc ggt ggt gac cat cca aag tgc atc      336
   Lys Lys Tyr Arg Glu Phe Gln Cys Gly Gly Asp His Pro Lys Cys Ile
               100                 105                 110
   tcg aac ctc ctc gtc tgc gac ggt gac aac gac tgt gac aat gga gct      384
   Ser Asn Leu Leu Val Cys Asp Gly Asp Asn Asp Cys Asp Asn Gly Ala
           115                 120                 125
   gat gag gct cgt tgt gat gtg ctc acc gag gct ggt agt agt tgg act      432
   Asp Glu Ala Arg Cys Asp Val Leu Thr Glu Ala Gly Ser Ser Trp Thr
       130                 135                 140
   ggt act gtg gtc tac gat cac tgc acc aag cgt cgc cca gag acc atg      480
   Gly Thr Val Val Tyr Asp His Cys Thr Lys Arg Arg Pro Glu Thr Met
   145                 150                 155                 160
   aag ctc agc atc aag agc gtg gat acc gta ccc ttc ttc acc acc cac      528
   Lys Leu Ser Ile Lys Ser Val Asp Thr Val Pro Phe Phe Thr Thr His
                   165                 170                 175
   ccc aag gtc cgc ggt acc gtg ctt atg gag aag cac acc aag gac tac      576
   Pro Lys Val Arg Gly Thr Val Leu Met Glu Lys His Thr Lys Asp Tyr
               180                 185                 190
   agc gag gtc atc aac gag ccg gtc tct ggc tac tgg agc agc gcc gat      624
   Ser Glu Val Ile Asn Glu Pro Val Ser Gly Tyr Trp Ser Ser Ala Asp
           195                 200                 205
   agg agc gcc gct atg ccc ccg gac agc gcc ggt cac ctt ggc ttt gtc      672
   Arg Ser Ala Ala Met Pro Pro Asp Ser Ala Gly His Leu Gly Phe Val
       210                 215                 220
   tgc atc ttc cac ggc cac gac cac gac acc tgc act ggt ctc ctc acc      720
   Cys Ile Phe His Gly His Asp His Asp Thr Cys Thr Gly Leu Leu Thr
   225                 230                 235                 240
   aag ggc aag gtc aca gat gcc tgc gcc gag ttc acc ttc cac agg gat      768
   Lys Gly Lys Val Thr Asp Ala Cys Ala Glu Phe Thr Phe His Arg Asp
                   245                 250                 255
   taa                                                                  771
<210> 12
   <211> 256
   <212> PRT
   <213> Arenicola marina
<400> 12
   Met Lys Ser Tyr Val Leu Val Cys Cys Leu Val Val Gly Ala Val Ala
   1               5                   10                  15
   Tyr Pro His Glu Val Met His His Ala Val Gly Ala Asn Arg Met Cys
               20                  25                  30
   Lys Cys Asp Ala Pro Ala Gly Asn Ala Glu Thr Ser Ala Asp Arg Glu
           35                  40                  45
   Gln Ser His Thr Leu Asp Glu Leu Thr His Gln Leu His Met Leu Gln
       50                  55                  60
   Gln Ala Tyr Asp Thr Gly Met Gly Arg Val Asp Asp Val Met Glu Asp
   65                  70                  75                  80
   Met Asp Asp Leu Ser His Arg Ile Ala Asp His Glu Lys Glu His Cys
                   85                  90                  95
   Lys Lys Tyr Arg Glu Phe Gln Cys Gly Gly Asp His Pro Lys Cys Ile
               100                 105                 110
   Ser Asn Leu Leu Val Cys Asp Gly Asp Asn Asp Cys Asp Asn Gly Ala
           115                 120                 125
   Asp Glu Ala Arg Cys Asp Val Leu Thr Glu Ala Gly Ser Ser Trp Thr
       130                 135                 140
   Gly Thr Val Val Tyr Asp His Cys Thr Lys Arg Arg Pro Glu Thr Met
   145                 150                 155                 160
   Lys Leu Ser Ile Lys Ser Val Asp Thr Val Pro Phe Phe Thr Thr His
                   165                 170                 175
   Pro Lys Val Arg Gly Thr Val Leu Met Glu Lys His Thr Lys Asp Tyr
               180                 185                 190
   Ser Glu Val Ile Asn Glu Pro Val Ser Gly Tyr Trp Ser Ser Ala Asp
           195                 200                 205
   Arg Ser Ala Ala Met Pro Pro Asp Ser Ala Gly His Leu Gly Phe Val
       210                 215                 220
   Cys Ile Phe His Gly His Asp His Asp Thr Cys Thr Gly Leu Leu Thr
   225                 230                 235                 240
   Lys Gly Lys Val Thr Asp Ala Cys Ala Glu Phe Thr Phe His Arg Asp
                   245                 250                 255
<210> 13
   <211> 376
   <212> ADN
   <213> Arenicola marina
<220>
   <221> CDS
   <222> (1)..(375)
   <223>
<400> 13
   gga cct ttg cag cgc ctc ctg gtc aag acc cag tgg aac aag gtg tac       48
   Gly Pro Leu Gln Arg Leu Leu Val Lys Thr Gln Trp Asn Lys Val Tyr
   1               5                   10                  15
   ggc acc agc aag gtc agg gac gag gcc gga cac gtc ctc tgg aag gct       96
   Gly Thr Ser Lys Val Arg Asp Glu Ala Gly His Val Leu Trp Lys Ala
               20                  25                  30
   att ttc gcc cag gat ccc gag acc cgg gct ctc ttc aag aga gtc aac      144
   Ile Phe Ala Gln Asp Pro Glu Thr Arg Ala Leu Phe Lys Arg Val Asn
           35                  40                  45
   ggt gac gac atc tac tct ccc gag ttc atg gct cac agc gcc cgt gtc      192
   Gly Asp Asp Ile Tyr Ser Pro Glu Phe Met Ala His Ser Ala Arg Val
       50                  55                  60
   ttg ggt ggc ctt gac att gcc atc tcc ctc ctc gac aac cag gct gac      240
   Leu Gly Gly Leu Asp Ile Ala Ile Ser Leu Leu Asp Asn Gln Ala Asp
   65                  70                  75                  80
   ctt gac gtc gcc ctg gct cac ctt cac gtg cag cac gta gaa agg cac      288
   Leu Asp Val Ala Leu Ala His Leu His Val Gln His Val Glu Arg His
                   85                  90                  95
   atc cca acc cgc tac ttc gat ctg ttc aag aac gcc ctg atg gag tat      336
   Ile Pro Thr Arg Tyr Phe Asp Leu Phe Lys Asn Ala Leu Met Glu Tyr
               100                 105                 110
   gcc ccc agc gcc ctg gga cgc tgc ttt gat aag gac gca t                376
   Ala Pro Ser Ala Leu Gly Arg Cys Phe Asp Lys Asp Ala
           115                 120                 125
<210> 14
   <211> 125
   <212> PRT
   <213> Arenicola marina
<400> 14
   Gly Pro Leu Gln Arg Leu Leu Val Lys Thr Gln Trp Asn Lys Val Tyr
   1               5                   10                  15
   Gly Thr Ser Lys Val Arg Asp Glu Ala Gly His Val Leu Trp Lys Ala
               20                  25                  30
   Ile Phe Ala Gln Asp Pro Glu Thr Arg Ala Leu Phe Lys Arg Val Asn
           35                  40                  45
   Gly Asp Asp Ile Tyr Ser Pro Glu Phe Met Ala His Ser Ala Arg Val
       50                  55                  60
   Leu Gly Gly Leu Asp Ile Ala Ile Ser Leu Leu Asp Asn Gln Ala Asp
   65                  70                  75                  80
   Leu Asp Val Ala Leu Ala His Leu His Val Gln His Val Glu Arg His
                   85                  90                  95
   Ile Pro Thr Arg Tyr Phe Asp Leu Phe Lys Asn Ala Leu Met Glu Tyr
               100                 105                 110
   Ala Pro Ser Ala Leu Gly Arg Cys Phe Asp Lys Asp Ala
           115                 120                 125
<210> 15
   <211> 288
   <212> ADN
   <213> Arenicola marina
<220>
   <221> CDS
   <222> (1)..(288)
   <223>
<400> 15
   tgc gga ccc ctt cag cgc ctg aag gtc aag cgc cag tgg gct gag gct       48
   Cys Gly Pro Leu Gln Arg Leu Lys Val Lys Arg Gln Trp Ala Glu Ala
   1               5                   10                  15
   tat gga agc gga aac agc agg gag gaa ttc gga cac ttc atc tgg tcc       96
   Tyr Gly Ser Gly Asn Ser Arg Glu Glu Phe Gly His Phe Ile Trp Ser
               20                  25                  30
   cat gtc ttc cag cac tcg cct gct gcc cgc gac atg ttc aag cgc gtc      144
   His Val Phe Gln His Ser Pro Ala Ala Arg Asp Met Phe Lys Arg Val
           35                  40                  45
   cgc ggt gac aac atc cac acc cca gca ttc atg gcc cac gcc acc cgt      192
   Arg Gly Asp Asn Ile His Thr Pro Ala Phe Met Ala His Ala Thr Arg
       50                  55                  60
   gtg ctc ggt gga ctc gac atg tgc att gcc ctt ctc gat gat gaa ccc      240
   Val Leu Gly Gly Leu Asp Met Cys Ile Ala Leu Leu Asp Asp Glu Pro
   65                  70                  75                  80
   gtt ctg aac acg cag ctc gct cat ctt gcc aag caa cac gaa acc cgt      288
   Val Leu Asn Thr Gln Leu Ala His Leu Ala Lys Gln His Glu Thr Arg
                   85                  90                  95
<210> 16
   <211> 96
   <212> PRT
   <213> Arenicola marina
<400> 16
   Cys Gly Pro Leu Gln Arg Leu Lys Val Lys Arg Gln Trp Ala Glu Ala
   1               5                   10                  15
   Tyr Gly Ser Gly Asn Ser Arg Glu Glu Phe Gly His Phe Ile Trp Ser
               20                  25                  30
   His Val Phe Gln His Ser Pro Ala Ala Arg Asp Met Phe Lys Arg Val
           35                  40                  45
   Arg Gly Asp Asn Ile His Thr Pro Ala Phe Met Ala His Ala Thr Arg
       50                  55                  60
   Val Leu Gly Gly Leu Asp Met Cys Ile Ala Leu Leu Asp Asp Glu Pro
   65                  70                  75                  80
   Val Leu Asn Thr Gln Leu Ala His Leu Ala Lys Gln His Glu Thr Arg
                   85                  90                  95
<210> 17
   <211> 360
   <212> ADN
   <213> Arenicola marina
<220>
   <221> CDS
   <222> (1)..(360)
   <223>
<400> 17
   aag gtg aag cac caa tgg gtg cag gtg tac agc ggc cat ggt tac gag      48
   Lys Val Lys His Gln Trp Val Gln Val Tyr Ser Gly His Gly Tyr Glu
   1               5                   10                  15
   cgt gag gcg ttc ggc aga gag gtc ttc ctc gag atg tac aac cag gca      96
   Arg Glu Ala Phe Gly Arg Glu Val Phe Leu Glu Met Tyr Asn Gln Ala
               20                  25                  30
   ccc aag gcc aag gac ctc ttc acc agg gtc agg ggc gag aac gtc ttc     144
   Pro Lys Ala Lys Asp Leu Phe Thr Arg Val Arg Gly Glu Asn Val Phe
           35                  40                  45
   tcc ccc gag ttc gga gcc cac atg gtc cgt gtg ctc gga ggt ctc gac     192
   Ser Pro Glu Phe Gly Ala His Met Val Arg Val Leu Gly Gly Leu Asp
       50                  55                  60
   atg tgc atc gct ctg ctg tcc gat gac acc gtc ctc aac gcc cag ctt     240
   Met Cys Ile Ala Leu Leu Ser Asp Asp Thr Val Leu Asn Ala Gln Leu
   65                  70                  75                  80
   gct cac ctc agc acg cag cac aag gac cgt gga atc ccc aac gag tac     288
   Ala His Leu Ser Thr Gln His Lys Asp Arg Gly Ile Pro Asn Glu Tyr
                   85                  90                  95
   ttc gat gtg gtg aag gtc gcc ctc atg aag gtc gtc ccc ggc cac gtt     336
   Phe Asp Val Val Lys Val Ala Leu Met Lys Val Val Pro Gly His Val
               100                 105                 110
   tca cac ttc gat atc ggc gcg tgg                                  360
   Ser His Phe Asp Ile Gly Ala Trp
           115                 120
<210> 18
   <211> 120
   <212> PRT
   <213> Arenicola marina
<400> 18
   Lys Val Lys His Gln Trp Val Gln Val Tyr Ser Gly His Gly Tyr Glu
   1               5                   10                  15
   Arg Glu Ala Phe Gly Arg Glu Val Phe Leu Glu Met Tyr Asn Gln Ala
               20                  25                  30
   Pro Lys Ala Lys Asp Leu Phe Thr Arg Val Arg Gly Glu Asn Val Phe
           35                  40                  45
   Ser Pro Glu Phe Gly Ala His Met Val Arg Val Leu Gly Gly Leu Asp
       50                  55                  60
   Met Cys Ile Ala Leu Leu Ser Asp Asp Thr Val Leu Asn Ala Gln Leu
   65                  70                  75                  80
   Ala His Leu Ser Thr Gln His Lys Asp Arg Gly Ile Pro Asn Glu Tyr
                   85                  90                  95
   Phe Asp Val Val Lys Val Ala Leu Met Lys Val Val Pro Gly His Val
               100                 105                 110
   Ser His Phe Asp Ile Gly Ala Trp
           115                 120
<210> 19
   <211> 390
   <212> ADN
   <213> Arenicola marina
<220>
   <221> CDS
   <222> (1)..(390)
   <223>
<400> 19
   tgt tgc agt ata gag gac cgc aag gag gtc cag acg ctg tgg agt gag      48
   Cys Cys Ser Ile Glu Asp Arg Lys Glu Val Gln Thr Leu Trp Ser Glu
   1               5                   10                  15
   atc tgg agt gcc cag ttc act ggt cgc cgt gtc cag gtt gcc cag gct      96
   Ile Trp Ser Ala Gln Phe Thr Gly Arg Arg Val Gln Val Ala Gln Ala
               20                  25                  30
   gtg ttc gag gac ctc ttc cgc cgc gac ccc gag tcc aag aac ctg ttc     144
   Val Phe Glu Asp Leu Phe Arg Arg Asp Pro Glu Ser Lys Asn Leu Phe
           35                  40                  45
   aag cgc gtc aat gtt gac gac atg aac agc ccc gaa ttc cac gct cac       192
   Lys Arg Val Asn Val Asp Asp Met Asn Ser Pro Glu Phe His Ala His
       50                  55                  60
   tgc atc cgt gtt gtc aac ggt ctt gac acc gtg atc ggt ctc ctt gac       240
   Cys Ile Arg Val Val Asn Gly Leu Asp Thr Val Ile Gly Leu Leu Asp
   65                  70                  75                  80
   gac ccc gac acc ctg aag tcc cag ctc gag cac ttg gcc cag cag cac       288
   Asp Pro Asp Thr Leu Lys Ser Gln Leu Glu His Leu Ala Gln Gln His
                   85                  90                  95
   aag gag cgt gat ggc atc cac aag acc cac ttc gac gag atg tcc cac       336
   Lys Glu Arg Asp Gly Ile His Lys Thr His Phe Asp Glu Met Ser His
               100                 105                 110
   gcc ttc ggc gcc gtc atg ccc cag gtc agc agc tgt ttc aac ccc gat       384
   Ala Phe Gly Ala Val Met Pro Gln Val Ser Ser Cys Phe Asn Pro Asp
           115                 120                 125
   gca tga                                                               390
   Ala
<210> 20
   <211> 129
   <212> PRT
   <213> Arenicola marina
<400> 20
   Cys Cys Ser Ile Glu Asp Arg Lys Glu Val Gln Thr Leu Trp Ser Glu
   1               5                   10                  15
   Ile Trp Ser Ala Gln Phe Thr Gly Arg Arg Val Gln Val Ala Gln Ala
               20                  25                  30
   Val Phe Glu Asp Leu Phe Arg Arg Asp Pro Glu Ser Lys Asn Leu Phe
           35                  40                  45
   Lys Arg Val Asn Val Asp Asp Met Asn Ser Pro Glu Phe His Ala His
       50                  55                  60
   Cys Ile Arg Val Val Asn Gly Leu Asp Thr Val Ile Gly Leu Leu Asp
   65                  70                  75                  80
   Asp Pro Asp Thr Leu Lys Ser Gln Leu Glu His Leu Ala Gln Gln His
                   85                  90                  95
   Lys Glu Arg Asp Gly Ile His Lys Thr His Phe Asp Glu Met Ser His
               100                 105                 110
   Ala Phe Gly Ala Val Met Pro Gln Val Ser Ser Cys Phe Asn Pro Asp
           115                 120                 125
   Ala
<210> 21
   <211> 20
   <212> ADN
   <213> artificial sequence
<220>
   <223> amorce PCR
<220>
   <221> misc_feature
   <222> (9)..(9)
   <223> A, G, C ou T
<220>
   <221> misc_feature
   <222> (12)..(-12)
   <223> A, G, C ou T
<400> 21
   gartgyggnc cnttrcarcg 20
<210> 22
   <211> 21
   <212> ADN
   <213> artificial sequence
<220>
   <223> amorce PCR
<400> 22
   ctcctctcct ctcctcttcc t 21
<210> 23
   <211> 17
   <212> ADN
   <213> artificial sequence
<220>
   <223> amorce PCR
<220>
   <221> misc_feature
   <222> (6)..(6)
   <223> A, G, C ou T
<220>
   <221> misc_feature
   <222> (12)..(12)
   <223> A, G, C ou T
<400> 23
   tgyggnathc tncarcg 17
<210> 24
   <211> 18
   <212> ADN
   <213> artificial sequence
<220>
   <223> amorce PCR
<220>
   <221> misc_feature
   <222> (6)..(6)
   <223> inosine
<220>
   <221> misc_feature
   <222> (12)..(12)
   <223> A, G, C ou T
<400> 24
   aargtnaarc anaactgg 18
<210> 25
   <211> 20
   <212> ADN
   <213> artificial sequence
<220>
   <223> amorce PCR
<400> 25
   tgytgyagya thgargaycg 20
<210> 26
   <211> 20
   <212> ADN
   <213> artificial sequence
<220>
   <223> amorce PCR
<220>
   <221> misc_feature
   <222> (6)..(6)
   <223> A, G, C ou T
<220>
   <221> misc_feature
   <222> (15)..(15)
   <223> A, G, C ou T
<400> 26
   aargtnatht tyggnagrga 20
<210> 27
   <211> 20
   <212> ADN
   <213> artificial sequence
<220>
   <223> amorce PCR
<220>
   <221> misc_feature
   <222> (15)..(15)
   <223> A, G, C ou T
<220>
   <221> misc_feature
   <222> (18)..(18)
   <223> A, G, C ou T
<400> 27
   garcaycart gyggnggnga 20
<210> 28
   <211> 21
   <212> ADN
   <213> artificial sequence
<220>
   <223> amorce PCR
<220>
   <221> misc_feature
   <222> (4)..(4)
   <223> A, G, C ou T
<220>
   <221> misc_feature
   <222> (7)..(7)
   <223> A, G, C ou T
<400> 28
   ccangcntcy ttrtcraagc a 21
<210> 29
   <211> 19
   <212> ADN
   <213> artificial sequence
<220>
   <223> amorce PCR
<220>
   <221> misc_feature
   <222> (2)..(2)
   <223> A, G, C ou T
<220>
   <221> misc_feature
   <222> (8)..(8)
   <223> A, G, C ou T
<220>
   <221> misc_feature
   <222> (11)..(11)
   <223> A, G, C ou T
<220>
   <221> misc_feature
   <222> (14)..(14)
   <223> A, G, C ou T
<400> 29
   antgyggncc nctncarcg 19
<210> 30
   <211> 18
   <212> ADN
   <213> artificial sequence
<220>
   <223> amorce PCR
<220>
   <221> misc_feature
   <222> (4)..(4)
   <223> A, G, C ou T
<220>
   <221> misc_feature
   <222> (7)..(7)
   <223> A, G, C ou T
<400> 30
   ccangcnccd atrtcraa 18
<210> 31
   <211> 20
   <212> ADN
   <213> artificial sequence
<220>
   <223> amorce PCR
<220>
   <221> misc_feature
   <222> (3)..(3)
   <223> A, G, C ou T
<220>
   <221> misc_feature
   <222> (6)..(6)
   <223> A, G, C ou T
<400> 31
   cangcnycrc trttraarca 20

## Description

La présente invention a pour objet un procédé de dissociation de la molécule d'hémoglobine extracellulaire d'Annélides, notamment d'*Arenicola marina,* ainsi que la caractérisation des chaînes protéiques constituant ladite molécule.

La présente invention a également pour objet la caractérisation des séquences nucléotidiques codant pour les chaînes protéiques susmentionnées, ainsi que le procédé de préparation de ces séquences nucléotidiques.

Le sang est un liquide complexe dont la fonction principale est de transporter l'oxygène et le gaz carbonique, afin d'assurer les processus respiratoires. C'est la molécule d'hémoglobine, que l'on trouve dans les hématies, qui assure cette fonction.

La molécule d'hémoglobine de mammifère est formée par l'assemblage de quatre chaînes polypeptidiques fonctionnelles semblables deux à deux (2 chaînes de globine de type α et 2 chaînes de globine de type β). Chacune de ces chaînes polypeptidiques possède la même structure tertiaire d'une molécule de myoglobine (Dickerson et Geis, 1983).

L'hème, site actif de l'hémoglobine, est un anneau protoporphyrinique tétrapyrrolique, contenant en son centre un unique atome de fer. L'atome de fer, fixateur de l'oxygène, établit 6 liaisons de coordinence : quatre avec les atomes d'azote de la porphyrine, une avec l'histidine proximale F8 et une avec la molécule d'oxygène lors de l'oxygénation de la globine.

On est actuellement confronté aux problèmes d'approvisionnement de sang, dus à la diminution des dons du sang par peur de contamination. Ainsi, la recherche de substituts sanguins s'est accélérée au cours des dernières années. On cherche à concevoir des substituts sanguins artificiels capables d'éliminer les risques de transmission de maladies infectieuses, mais également à s'affranchir des problèmes de compatibilité des groupes sanguins.

Jusqu'à présent, les principales voies de recherche concernent la synthèse de produits chimiques d'une part (Clark et Gollan, 1966) et la synthèse de produits biologiques d'autre part (Chang, 1957 ; Chang, 1964).

En ce qui concerne la première voie de recherche, on a utilisé les perfluorocarbones (PFC). Les PFC sont des produits chimiques capables de transporter l'oxygène et ils peuvent dissoudre une grande quantité de gaz, comme l'oxygène et le dioxyde de carbone.

Actuellement, on essaye de produire des émulsions de ces produits qui pourraient être dispersés dans le sang d'une façon plus efficace (Reiss, 1991 ; Reiss, 1994 ; Goodin et al., 1994).

L'avantage des PFC demeure dans leur capacité oxyphorique directement proportionnelle à la quantité d'oxygène se trouvant dans les poumons. Par ailleurs, en raison de l'absence de membrane à traverser, les PFC peuvent transporter l'oxygène plus rapidement vers les tissus. Toutefois, on ne connaît pas les effets à long terme de la rétention de ces produits dans l'organisme. Lorsque ces produits ont été utilisés pour la première fois comme substitut sanguin dans les années 1960 chez la souris (Clark et Gollan, 1966 ; Geyer et al., 1966 ; Sloviter et Kamimoto, 1967), les effets secondaires ont été très importants. Les PFC n'étaient pas éliminés de la circulation d'une façon satisfaisante et s'accumulaient dans les tissus de l'organisme, ce qui provoquait des oedèmes.

Dans les années 1980, on a testé une nouvelle version de PFC en phase clinique. Mais les problèmes de stockage, de coût financier, d'effets secondaires non négligeables et la faible efficacité de ce composé ont empêché l'extension de sa commercialisation (Naito, 1978 ; Mitsuno et Naito, 1979 ; Mitsuno et Ohyanagi, 1985).

Récemment, on a mis au point une nouvelle génération de PFC (PFBO perfluorooctylbromide). Un nouveau produit (Reiss, 1991) est en cours de test clinique aux Etats-Unis, mais on a déjà constaté qu'une augmentation de la quantité d'oxygène dans le sang peut engendrer une accumulation d'oxygène dans les tissus, ce qui est dangereux pour l'organisme (formation d'oxygène radicalaire de type superoxyde).

Ainsi, malgré les progrès réalisés au fur et à mesure, les effets secondaires de ces composés sont encore trop importants pour être commercialisés à grande échelle.

En ce qui concerne la deuxième voie de recherche, des chercheurs ont travaillé sur la mise au point de substituts sanguins en modifiant la structure de l'hémoglobine naturelle (Chang, 1957 ; Chang, 1997). Pour obtenir un substitut sanguin de type hémoglobine modifiée, on utilise des hémoglobines synthétisées par des microorganismes génétiquement modifiés, ou d'origine humaine ou animale, notamment la molécule d'hémoglobine bovine. En effet, l'hémoglobine bovine est légèrement différente de l'hémoglobine humaine sur le plan immunologique, mais elle transporte plus facilement l'oxygène vers les tissus. Néanmoins, le risque de contamination virale ou de type encéphalopathie spongiforme reste toujours important.

Pour être fonctionnelle, l'hémoglobine doit être en contact avec un effecteur allostérique le 2,3-diphosphoglycérate (2,3-DPG), présent uniquement à l'intérieur des globules rouges (Dickerson et Geis, 1983). De plus, sans le 2,3-DPG et d'autres éléments présents dans les globules rouges comme la méthémoglobine réductase, l'hémoglobine subit un processus d'auto-oxydation et perd sa capacité à transporter de l'oxygène ou du dioxyde de carbone.

On peut éliminer ces processus en modifiant la structure de l'hémoglobine, et plus précisément en stabilisant les liaisons faibles de la molécule tétramérique entre les deux dimères α et β (Chang, 1971). De nombreuses modifications ont été testées : liaison covalente entre deux chaînes α, entre deux chaînes β ou encore entre α et β (Payne, 1973 ; Bunn et Jandl, 1968).

On a également tenté de polymériser les molécules tétramériques ou de les conjuguer avec un polymère nommé polyéthylène glycol (PEG)(Nho et al., 1992). Ces modifications ont pour conséquence de stabiliser la molécule et d'augmenter sa taille, empêchant son élimination par les reins.

On a beaucoup étudié les Annélides pour leur hémoglobine extracellulaire (Terwilliger, 1992 ; Lamy et al., 1996). Ces molécules d'hémoglobine extracellulaire sont présentes chez les trois classes d'Annélides : Polychètes, Oligochètes et Achètes et même chez les Vestimentifères. Ce sont des biopolymères géants, constitués d'environ 200 chaînes polypeptidiques appartenant à 6 ou 8 types différents que l'on regroupe généralement en deux catégories. La première catégorie, comptant 144 à 192 éléments, regroupe les chaînes polypeptidiques dites "fonctionnelles" portant un site actif et capables de lier réversiblement l'oxygène ; ce sont des chaînes de type globine dont les masses sont comprises entre 15 et 18 kDa et qui sont très similaires aux chaînes de type α et β de vertébrés. La deuxième catégorie, comptant 36 à 42 éléments, regroupe les chaînes polypeptidiques dites de "structure" possédant peu ou pas de site actif mais permettant l'assemblage des douzièmes.

Les premières images obtenues sur des hémoglobines extracellulaires d'Arénicole (Roche et al., 1960) ont révélé des structures hexagonales. Chaque molécule: d'hémoglobine est constituée de deux hexagones superposés (Levin, 1963 ; Roche, 1965) que l'on dit en "bicouche hexagonale" (hexagonal bilayer) et chaque hexagone est lui-même formé par l'assemblage de six éléments en forme de goutte d'eau (Van Bruggen et Weber, 1974 ; Kapp et Crewe, 1984), nommés structure globulaire creuse (hollow globular structure)(De Haas et al., 1996) ou "douzième". La molécule native est formée de douze de ces sous-unités, d'une masse moléculaire d'environ 250 kDa.

Ainsi, le brevet français n°2 809 624 concerne l'utilisation comme substitut sanguin d'hémoglobine extracellulaire *d'Arenicola marina,* un Annélide Polychète de l'écosystème intertidal, ledit substitut sanguin permettant d'éliminer les problèmes de pénurie de dons.

Bien que l'architecture globale de l'hémoglobine de *Arenicola marina* soit connue, notamment grâce à son étude quaternaire fine par spectrométrie de masse (Zal et al., 1997), les séquences primaires des différentes chaînes protéiques qui la composent ne le sont pas.

Ainsi, la présente invention a pour but de fournir les séquences protéiques qui composent la molécule d'hémoglobine *d'Arenicola marina.*

Un autre but de la présente invention consiste à fournir les premières étapes de la synthèse *in vitro* d'hémoglobine extracellulaire *d'Arenicola marina* afin d'élaborer un substitut sanguin au moyen de procédés de biochimie et de biologie moléculaire.

Un autre but de la présente invention consiste à fournir un procédé de préparation de la molécule d'hémoglobine, éventuellement simplifiée, par génie génétique, et ce afin notamment d'augmenter le stock de cette molécule dans le cadre d'une utilisation comme substitut sanguin.

La présente invention concerne un procédé d'obtention des chaînes protéiques constituant la molécule d'hémoglobine extracellulaire d'*Arenicola marina,*

ledit procédé étant caractérisé en ce qu'il comprend une étape de mise en présence d'un échantillon d'hémoglobine extracellulaire d'*Arenicola marina* avec au moins un agent dissociant, et un agent réducteur, qui est un mélange constitué de dithiothréitol (DTT) ou de chlorhydrate de tris(2-carboxyéthyl)phosphine (TCEP) ou de bêta-mercaptoéthanol et d'un tampon de dissociation, pendant un temps suffisant pour séparer les chaînes protéiques les unes des autres.

Par "hémoglobine extracellulaire", on désigne une hémoglobine non contenue dans les cellules et dissoute dans le sang.

L'expression "dissociation" désigne un traitement chimique capable de rompre les interactions faibles (hydrophobes, électrostatiques, hydrogènes...).

Par "tampon de dissociation", on désigne un liquide contenant un tampon permettant d'ajuster le pH et contenant des agents dissociants.

L'expression "agent dissociant" désigne un composé chimique capable de rompre les interactions faibles (hydrophobes, électrostatiques, hydrogènes...). Ledit agent dissociant est notamment choisi parmi : les ions hydroxydes, l'urée ou des ions hétéropolytungstates ou des sels de guanidinium ou du SDS (sodium dodécyl sulfate).

L'expression "réduction" désigne un traitement chimique capable de rompre les interactions fortes telles que les ponts bisulfures.

L'expression "agent réducteur" désigne un composé chimique capable de rompre les interactions fortes telles que les ponts disulfures.

Les dix chaînes protéiques constituant la molécule d'hémoglobine extracellulaire d'*Arenicola marina* comprennent 8 chaînes de type globine et 2 chaînes de type structure.

On rappelle que l'hémoglobine extracellulaire de *Arenicola marina* d'une masse de 3648 ± 24 kDa est constituée de 198 chaînes polypeptidiques appartenant à 10 types différents regroupés en deux catégories :
- la première (156 chaînes) regroupe des chaînes polypeptidiques dites "fonctionnelles" portant un site actif capable de lier réversiblement l'oxygène ; ce sont des chaînes de type globine dont les masses sont comprises entre 15 et 18 kDa ; ces chaînes sont très similaires aux chaînes de types α et β des vertébrés ; et
- la deuxième (42 chaînes) regroupe des chaînes polypeptidiques dites de "structure" (ou "linker") possédant peu ou pas de site actif mais permettant l'assemblage des dodécamères ; ces chaînes ont des masses moléculaires comprises entre 22 et 27 kDa.

La présente invention concerne un procédé de dissociation de la molécule d'hémoglobine extracellulaire d'Arenicola marina, permettant l'obtention des chaînes protéiques constituant ladite molécule,
ledit procédé étant caractérisé en ce qu'il comprend une étape de mise en présence d'un échantillon d'hémoglobine extracellulaire d'Arenicola marina avec un mélange constitué de dithiothréitol (DTT) et d'un tampon de dissociation, pendant environ une heure à trois semaines.

Un procédé de dissociation avantageux de l'invention est caractérisé en ce que le tampon de dissociation comprend un agent tamponnant à une concentration comprise d'environ 0,05 M à environ 0,1 M, notamment du Trisma (tris[hydroxyméthyl]aminométhane), de l'hepes, du phosphate de sodium, du borate de sodium, du bicarbonate d'ammonium ou de l'acétate d'ammonium, et 0 à 10 mM d'EDTA ajusté à un pH compris d'environ 5 à environ 12, et de préférence d'environ 7,5 à 12, le tout étant notamment ajusté à un pH compris d'environ 2 à 12, et de préférence d'environ 5 à 12.

Ledit tampon de dissociation comprend de l'EDTA à une concentration d'environ 1 mM ajusté à un pH d'environ 10, notamment avec une solution de soude 2N.

Selon un mode de réalisation alternatif, le procédé de l'invention est caractérisé en ce que les chaînes protéiques constituant ladite molécule sont obtenues par la réduction de quatre sous-unités par un agent réducteur, par exemple en présence de DTT, lesdites sous-unités étant elles-mêmes obtenues par mise en présence d'un échantillon d'hémoglobine extracellulaire d'*Arenicola marina* avec un tampon de dissociation.

La molécule native se dissocie en sous-unités sous l'action d'agents dissociants non réducteurs. Il n'y a donc pas rupture des liaisons covalentes. Toutefois, après l'action d'un agent réducteur (coupure des liaisons covalentes), les sous-unités sont réduites en chaînes polypeptidiques (chaînes protéiques constituées de l'assemblage d'acides aminés).

Les 4 sous-unités susmentionnées sont donc : les monomères, les dimères, les trimères et les dodécamères.

Les monomères sont des chaînes de globine.

Les dimères sous forme homo et héterodimères sont des chaînes de structure.

Les trimères sont des assemblages covalents de trois chaînes de globine.

Les dodecamères sont constitués de 12 chaînes protéiques ; par exemple : 3 trimères + 3 monomères, 2 trimères + 6 monomères, 1 trimère + 9 monomères.

On peut donc obtenir les chaînes protéiques soit en une seule étape par réduction directe de l'hémoglobine extracellulaire d'*Arenicola marina,* soit en deux étapes, l'une consistant en la dissociation de l'hémoglobine extracellulaire d'*Arenicola marina* en 4 sous-unités et l'autre en la réduction desdites 4 sous-unités en chaînes protéiques.

La présente invention concerne également un procédé de dissociation tel que défini ci-dessus, caractérisé en ce que les agents dissociants utilisés pour obtenir les 4 sous-unités susmentionnées sont les suivants :
- une solution tampon de dissociation comprenant : 0,1 M de base Trisma (tris[hydroxyméthyl]aminométhane) et 0 à 10 mM d'EDTA ajusté à un pH compris d'environ 5 à environ 12, et de préférence d'environ 7,5 à environ 12, et
- une solution d'urée dont la concentration est comprise d'environ 0,1 M à environ 8 M, et est notamment égale à 3 M.

La présente invention concerne également un procédé de dissociation tel que défini ci-dessus, caractérisé en ce que les agents dissociants pour obtenir les 4 sous-unités sont les suivants :
- une solution tampon de dissociation comprenant 0,1 M de base Trisma et 1 mM d'EDTA ajusté à pH 10, et
- 3 M d'urée.

La présente invention concerne également un procédé de dissociation et DE réduction tel que défini ci-dessus, caractérisé en ce que les agents dissociants et réducteurs utilisés pour obtenir les chaînes protéiques sont les suivants :
- une solution tampon de dissociation comprenant : 0,1 M de base Trisma (tris[hydroxyméthyl]aminométhane) à un pH compris d'environ 8 à environ 9, et
- une solution d'urée dont la concentration est comprise d'environ 4 M à environ 8 M, et est notamment égale à 8 M, et
- 1 à 10% DTT
ou
- une solution tampon de dissociation comprenant : 0,1 M de bicarbonate d'ammonium à un pH compris d'environ 8 à environ 9, et
- 1 à 10% DTT

Le procédé de dissociation et de réduction de l'invention permet d'obtenir une composition contenant le mélange des chaînes protéiques constituant la molécule d'hémoglobine extracellulaire d'*Arenicola marina.*

La présente invention concerne également un procédé de préparation de couples d'amorces à partir des chaînes protéiques telles qu'obtenues selon le procédé tel que défini ci-dessus, ledit procédé étant caractérisé en ce qu'il comprend les étapes suivantes :
- l'isolement de chacune des chaînes protéiques constituant la molécule d'hémoglobine telles qu'obtenues selon le procédé tel que défini ci-dessus,
- le microséquençage de chacune des chaînes protéiques isolées susmentionnées par spectrométrie de masse et séquençage d'Edman, afin d'obtenir une microséquence correspondant à chacune des séquences constituées de 5 à 20 acides aminés, et
- la détermination des amorces dégénérées à partir des microséquences susmentionnées.

La première étape d'isolement des chaînes protéiques est notamment effectuée par chromatographie liquide en phase inverse et gel bidimensionnel à partir du mélange susmentionné comprenant les chaînes protéiques constituant la molécule d'hémoglobine telles qu'obtenues selon le procédé de dissociation et de réduction de l'invention.

L'expression "microséquence" désigne des fragments de séquences protéiques.

Les microséquences susmentionnés peuvent provenir à la fois des extrémités C- et N-terminales mais aussi de séquences internes.

Les chaînes protéiques peuvent être obtenues par chromatographie liquide en phase inverse ou à partir de gel 2D à partir d'hémoglobine purifiée d'*Arenicola marina.* Chaque pic ou tache a été découpé et digéré par une protéase. Les peptides ainsi obtenus ont été extraits des gels et séparés par capLC (chromatographie liquide capillaire). Les fragments sont ensuite analysés par spectrométrie de masse. D'autre part, chaque pic isolé par phase inverse a été analysé par séquençage d'Edman.

L'expression "amorces dégénérées" désigne des séquences nucléotidiques obtenues à partir de fragments de séquences protéiques. On parle d'amorces dégénérées en raison de la dégénérescence du code génétique (plusieurs codons pour 1 acide aminé).

La dernière étape de détermination des couples d'amorces dégénérées correspond à leur synthèse.

Cette étape permet d'obtenir à la fois des amorces sens et des amorces antisens.

La présente invention concerne également des couples d'amorces tels qu'obtenus selon le procédé tel que défini ci-dessus, lesdits couples étant notamment les suivants :
a) *Amorce sens :* GAR TGY GGN CCN TTR CAR CG (SEQ ID NO : 21)
   *Amorce antisens :* CTC CTC TCC TCT CCT CTT CCT (SEQ ID NO : 22)
b) *Amorce sens :* TGY GGN ATH CTN CAR CG (SEQ ID NO : 23)
   *Amorce antisens :* CTC CTC TCC TCT CCT CTT CCT (SEQ NO : 22)
c) *Amorce sens :* AAR GTI AAR CAN AAC TGG (SEQ ID NO : 24)
   *Amorce antisens :* CTC CTC TCC TCT CCT CTT CCT (SEQ ID NO : 22)
d) *Amorce sens :* TGY TGY AGY ATH GAR GAY CG (SEQ ID NO : 25)
   *Amorce antisens :* CTC CTC TCC TCT CCT CTT CCT (SEQ ID NO : 22)
e) *Amorce sens :* AAR GTN ATH TTY GGN AGR GA (SEQ ID NO : 26)
   *Amorce antisens :* CTC CTC TCC TCT CCT CTT CCT (SEQ ID NO : 22)
f) *Amorce sens :* GAR CAY CAR TGY GGN GGN GA (SEQ ID NO : 27)
   *Amorce antisens :* CTC CTC TCC TCT CCT CTT CCT (SEQ ID NO : 22) où :
   R représente A ou G,
   Y représente C ou T,
   N représente A, G, C ou T,
   I représente l'inosine,
   H représente A, C ou T.

La présente invention concerne également des couples d'amorces tels qu'obtenus selon le procédé tel que défini ci-dessus, lesdits couples étant notamment les suivants :
a) *Amorce sens* : GAR TGY GGN CCN TTR CAR CG SEQ ID NO : 21
   *Amorce antisens* : CCA NGC NTC YTT RTC RAA GCA SEQ ID NO : 28
b) *Amorce sens* : AN TGY GGN CCN CTN CAR CG SEQ ID NO : 29
   *Amorce antisens* : CCA NGC NTC YTT RTC RAA GCA SEQ ID NO : 28
c) *Amorce sens* : AAR GTI AAR CAN AAC TGG SEQ ID NO : 24
   *Amorce antisens* : CCA NGC NCC DAT RTC RAA SEQ NO : 30
d) *Amorce sens* : TGY TGY AGY ATH GAR GAY CG SEQ ID NO : 25
   *Amorce antisens* : CA NGC NYC RCT RTT RAA RCA SEQ ID NO : 31 où :
   R représente A ou G,
   Y représente C ou T,
   N représente A, G, C ou T,
   I représente l'inosine,
   D représente A, G ou T,
   H représente A, C ou T.

La présente invention concerne également un procédé de préparation de séquences nucléotidiques codant pour les chaînes protéiques constituant la molécule d'hémoglobine *d'Arenicola marina,* à partir des amorces telles qu'obtenues selon le procédé tel que défini ci-dessus, ledit procédé étant caractérisé en ce qu'il correspond à un procédé d'amplification en chaîne par polymérase (PCR), comprenant une répétition d'au moins 30 fois du cycle constitué par les étapes suivantes :
* la dénaturation, par chauffage, de l'ADNc monocaténaire codant pour une des chaînes protéiques constituant la molécule d'hémoglobine d'*Arenicola marina,* de façon à dénaturer d'éventuelles structures secondaires et des reliquats d'ARN, ledit ADNc étant obtenu à partir d'ARNm, cette étape permettant d'obtenir des brins d'ADNc monocaténaires dénaturés,
* l'hybridation des couples d'amorces tels qu'obtenus par le procédé tel que défini ci-dessus aux brins d'ADNc monocaténaires dénaturés susmentionnés à une température adéquate, pour obtenir des amorces hybridées, et
* la synthèse du brin complémentaire de l'ADNc par une polymérase à une température adéquate, à partir des amorces hybridées telles qu'obtenues à l'étape précédente.

L'ADNc codant pour les chaînes protéiques susmentionnées est obtenu à partir d'ARNm, ledit ARNm étant obtenu par purification à partir d'ARN totaux extraits sur des Arénicoles juvéniles en pleine croissance, lesdits Arénicoles juvéniles présentant un taux élevé de transcription des différents ARN messagers.

Si le cycle susmentionné est répété moins de 30 fois, l'amplification de l'ADN est réduite.

L'expression "éventuelles structures secondaires" désigne des appariements anarchiques entre deux séquences d'ADNc.

L'expression "dénaturation par chauffage de l'ADNc" désigne la rupture des appariements anarchiques entre deux séquences d'ADNc.

L'expression "hybridation à une température adéquate" désigne la reconnaissance par les amorces de leurs séquences complémentaires sur l'ADN matrice.

Selon un mode de réalisation avantageux, le procédé de préparation de séquences nucléotidiques selon l'invention est caractérisé en ce que :
- la première étape dudit procédé est une étape de dénaturation de l'ADNc codant pour une des chaînes protéiques constituant la molécule d'hémoglobine d'*Arenicola marina* d'environ 10 secondes à environ 5 minutes à une température comprise d'environ 90°C à environ 110°C,
- le cycle, répété environ 30 à 40 fois, comprend les étapes suivantes :
   * une étape de dénaturation de l'ADNc codant pour une des chaînes protéiques constituant la molécule d'hémoglobine d'*Arenicola marina* d'environ 10 secondes à environ 5 minutes, à une température comprise d'environ 90°C à environ 110°C,
   * une étape d'hybridation des couples d'amorces de l'invention aux brins d'ADNc monocaténaires susmentionnés pour obtenir des amorces hybridées, d'environ 20 secondes à environ 2 minutes, à une température comprise d'environ 50°C à environ 60°C, de préférence d'environ 50°C à environ 56°C,
   * une étape d'élongation des amorces hybridées telles qu'obtenues précédemment par une polymérase d'environ 20 secondes à environ 1 minute et 30 secondes, à une température comprise d'environ 70°C à environ 75°C, et
- la dernière étape du procédé est une étape d'élongation des amorces hybridées telles qu'obtenues précédemment par une polymérase d'environ 5 minutes à environ 15 minutes à une température comprise d'environ 70°C à environ 75°C.

La réaction de PCR du procédé de l'invention est notamment effectuée en présence d'ADNc (5 à 20 ng), d'amorce sens (100 ng) et antisens (100 ng), de dNTP (200 µM final), de MgCl₂ (2 mM final), de tampon PCR (fourni avec la polymérase) (1 X final), de la Taq polymérase (1 unité) et d'eau (25 µl qsp).

Le procédé de préparation de séquences nucléotidiques susmentionné permet d'obtenir des séquences codantes partielles.

Une fois les séquences codantes partielles obtenues grâce aux expériences précédentes, l'amplification et le séquençage de la totalité de la séquence codante des ADNc de globines et du linker sont réalisés par 5' RACE (Rapid Amplification cDNA Ends) PCR et selon les recommandations protocolaires de la fiche technique fournie par le fournisseur (5'/3' RACE kit, Roche).

La présente invention concerne également un procédé de préparation tel que défini ci-dessus, caractérisé en ce que les couples d'amorces utilisés sont tels que définis précédemment.

Un procédé de préparation particulièrement avantageux selon l'invention est un procédé de préparation de séquences nucléotidiques tel que défini ci-dessus, caractérisé en ce que le couple d'amorces utilisé est : (GAR TGY GGN CCN TTR CAR CG ; CCA NGC NTC YTT RTC RAA GCA) ou (GAR TGY GGN CCN TTR CAR CG ; CTC CTC TCC TCT CCT CTT CCT), R, Y et N étant tels que définis ci-dessus,
ledit procédé étant caractérisé en ce que :
- la première étape du procédé est une étape de dénaturation de l'ADNc codant pour les chaînes protéiques constituant la molécule d'hémoglobine d'*Arenicola marina,* de 4 minutes à une température égale à 95°C,
- le cycle, répété 35 fois, comprend les étapes suivantes :
   * une étape de dénaturation de l'ADNc codant pour une des chaînes protéiques constituant la molécule d'hémoglobine d'*Arenicola marina,* de 30 secondes à une température égale à 95°C,
   * une étape d'hybridation des couples d'amorces de l'invention aux brins d'ADNc monocaténaires susmentionnés pour obtenir des amorces hybridées, de 30 secondes à une température égale à 56°C,
   * une étape d'élongation des amorces hybridées telles qu'obtenues précédemment par une polymérase de 40 secondes à une température égale à 72°C, et
- la dernière étape du procédé est une étape d'élongation des amorces hybridées telles qu'obtenues précédemment par une polymérase de 10 minutes à une température égale à 72°C,
afin d'obtenir la séquence nucléotidique SEQ ID NO : 13,
ledit procédé comprenant éventuellement une étape supplémentaire de 5' RACE PCR pour obtenir la séquence nucléotidique SEQ ID NO : 1.

La séquence partielle SEQ ID NO : 13 a ensuite été complétée par des expériences 5'RACE PCR comme expliqué ci-dessus. La séquence nucléotidique SEQ ID NO: 13 est une nouvelle séquence nucléotidique codant pour une chaîne protéique correspondant à une chaîne de globine, notée A2a. SEQ ID NO : 13 comprend 376 nucléotides.

La séquence nucléotidique SEQ ID NO : 1 (du codon initiateur au codon Stop, c'est à dire la séquence transcrite et traduite qui correspond à un monomère de globine fonctionnel) est une nouvelle séquence nucléotidique codant pour une chaîne protéique correspondant à la chaîne de globine susmentionnée, notée A2a. SEQ ID NO : 1 comprend 474 nucléotides.

Un procédé de préparation particulièrement avantageux selon l'invention est un procédé de préparation de séquences nucléotidiques tel que défini ci-dessus, caractérisé en ce que le couple d'amorces utilisé est le suivant : (AN TGY GGN CCN CTN CAR CG ; CCA NGC NTC YTT RTC RAA GCA), N, Y et R étant tels que définis ci-dessus,
ledit procédé étant caractérisé en ce que :
- la première étape du procédé est une étape de dénaturation de l'ADNc codant pour les chaînes protéiques constituant la molécule d'hémoglobine d'*Arenicola marina,* de 4 minutes à une température égale à 95°C,
- le cycle, répété 35 fois, comprend les étapes suivantes :
   * une étape de dénaturation de l'ADNc codant pour une des chaînes protéiques constituant la molécule d'hémoglobine d'*Arenicola marina,* de 30 secondes à une température égale à 95°C,
   * une étape d'hybridation des couples d'amorces de l'invention aux brins d'ADNc monocaténaires susmentionnés pour obtenir des amorces hybridées, de 30 secondes à une température égale à 52°C,
   * une étape d'élongation des amorces hybridées telles qu'obtenues précédemment par une polymérase de 40 secondes à une température égale à 72°C, et
- la dernière étape du procédé est une étape d'élongation des amorces hybridées telles qu'obtenues précédemment par une polymérase de 10 minutes à une température égale à 72°C,
afin d'obtenir la séquence nucléotidique SEQ ID NO : 15.

La séquence nucléotidique SEQ ID NO : 15 est une nouvelle séquence nucléotidique codant pour une chaîne protéique correspondant à une chaîne de globine, notée A2b. SEQ ID NO : 15 comprend 288 nucléotides.

Un procédé de préparation particulièrement avantageux selon l'invention est un procédé de préparation de séquences nucléotidiques tel que défini ci-dessus, caractérisé en ce que le couple d'amorces utilisé est le suivant : (TGY GGN ATH CTN CAR CG ; CTC CTC TCC TCT CCT CTT CCT), N, Y et R étant tels que définis ci-dessus,
ledit procédé étant caractérisé en ce que :
- la première étape du procédé est une étape de dénaturation de 4 minutes à une température égale à 95°C,
- le cycle, répété 35 fois, comprend les étapes suivantes :
   * une étape de dénaturation de 30 secondes à une température égale à 95°C,
   * une étape d'hybridation de 30 secondes à une température égale à 53°C,
   * une étape d'élongation de 40 secondes à une température égale à 72°C, et
- la dernière étape du procédé est une étape d'élongation de 10 minutes à une température égale à 72°C,
afin d'obtenir la séquence nucléotidique SEQ ID NO : 15,
ledit procédé comprenant éventuellement une étape supplémentaire de 5' RACE PCR pour obtenir la séquence nucléotidique SEQ ID NO : 3.

La séquence partielle SEQ ID NO : 15 a ensuite été complétée par des expériences 5'RACE PCR comme expliqué ci-dessus.

La séquence nucléotidique SEQ ID NO : 3 (du codon initiateur au codon Stop, c'est à dire la séquence transcrite et traduite qui correspond à un monomère de globine fonctionnel) est une nouvelle séquence nucléotidique codant pour une chaîne protéique correspondant à la chaîne de globine susmentionnée, notée A2b. SEQ ID NO : 3 comprend 477 nucléotides.

Un procédé de préparation particulièrement avantageux selon l'invention est un procédé de préparation de séquences nucléotidiques tel que défini ci-dessus, caractérisé en ce que le couple d'amorces utilisé est : (AAR GTI AAR CAN AAC TGG ; CCA NGC NCC DAT RTC RAA) ou (AAR GTI AAR CAN AAC TGG ; CTC CTC TCC TCT CCT CTT CCT), R, I, N et D étant tels que définis ci-dessus,
ledit procédé étant caractérisé en ce que :
- la première étape du procédé est une étape de dénaturation de l'ADNc codant pour chacune des chaînes protéiques constituant la molécule d'hémoglobine d'*Arenicola marina,* de 4 minutes à une température égale à 95°C,
- le cycle, répété 35 fois, comprend les étapes suivantes :
   * une étape de dénaturation de l'ADNc codant pour une des chaînes protéiques constituant la molécule d'hémoglobine d'*Arenicola marina,* de 1 minute à une température égale à 95°C,
   * une étape d'hybridation des couples d'amorces de l'invention aux brins d'ADNc monocaténaires susmentionnés pour obtenir des amorces hybridées, de 1 minute à une température égale à 50°C,
   * une étape d'élongation des amorces hybridées telles qu'obtenues précédemment par une polymérase de 1 minute et 30 secondes à une température égale à 72°C, et
- la dernière étape du procédé est une étape d'élongation des amorces hybridées telles qu'obtenues précédemment par une polymérase de 10 minutes à une température égale à 72°C,
afin d'obtenir la séquence nucléotidique SEQ ID NO : 17,
ledit procédé comprenant éventuellement une étape supplémentaire de 5' RACE PCR pour obtenir la séquence nucléotidique SEQ ID NO : 5.

La séquence partielle SEQ ID NO : 17 a ensuite été complétée par des expériences 5'RACE PCR comme expliqué ci-dessus. La séquence nucléotidique SEQ ID NO: 17 est une nouvelle séquence nucléotidique codant pour une chaîne protéique correspondant à une chaîne de globine, notée A1. SEQ ID NO : 17 comprend 360 nucléotides.

La séquence nucléotidique SEQ ID NO : 5 (du codon initiateur au codon Stop, c'est à dire la séquence transcrite et traduite qui correspond à un monomère de globine fonctionnel) est une nouvelle séquence nucléotidique codant pour une chaîne protéique correspondant à la chaîne de globine susmentionnée, notée A1. SEQ ID NO: 5 comprend 474 nucléotides.

Un procédé de préparation particulièrement avantageux selon l'invention est un procédé de préparation de séquences nucléotidiques tel que défini ci-dessus, caractérisé en ce que le couple d'amorces utilisé est le suivant : (TGY TGY AGY ATH GAR GAY CG ; CA NGC NYC RCT RTT RAA RCA) ou (TGY TGY AGY ATH GAR GAY CG ; CTC CTC TCC TCT CCT CTT CCT), Y, H, R et N étant tels que définis ci-dessus,
ledit procédé étant caractérisé en ce que :
- la première étape du procédé est une étape de dénaturation de l'ADNc codant pour les chaînes protéiques constituant la molécule d'hémoglobine d'*Arenicola marina,* de 4 minutes à une température égale à 95°C,
- le cycle, répété 35 fois, comprend les étapes suivantes :
   * une étape de dénaturation de l'ADNc codant pour une des chaînes protéiques constituant la molécule d'hémoglobine d'*Arenicola marina,* de 30 secondes à une température égale à 95°C,
   * une étape d'hybridation des couples d'amorces de l'invention aux brins d'ADNc monocaténaires susmentionnés pour obtenir des amorces hybridées, de 40 secondes à une température égale à 52°C,
   * une étape d'élongation des amorces hybridées telles qu'obtenues précédemment par une polymérase de 30 secondes à une température égale à 72°C, et
- la dernière étape du procédé est une étape d'élongation des amorces hybridées telles qu'obtenues précédemment par une polymérase de 10 minutes à une température égale à 72°C,
afin d'obtenir la séquence nucléotidique SEQ ID NO : 19,
ledit procédé comprenant éventuellement une étape supplémentaire de 5' RACE PCR pour obtenir la séquence nucléotidique SEQ ID NO : 7.

La séquence partielle SEQ ID NO : 19 a ensuite été complétée par des expériences 5'RACE PCR comme expliqué ci-dessus. La séquence nucléotidique SEQ ID NO : 19 est une nouvelle séquence nucléotidique codant pour une chaîne protéique correspondant à une chaîne de globine, notée B2. SEQ ID NO : 19 comprend 390 nucléotides.

La séquence nucléotidique SEQ ID NO : 7 (du codon initiateur au codon Stop, c'est à dire la séquence transcrite et traduite qui correspond à un monomère de globine fonctionnel) est une nouvelle séquence nucléotidique codant pour une chaîne protéique correspondant à la chaîne de globine susmentionnée, notée B2. SEQ ID NO : 7 comprend 498 nucléotides.

Un procédé de préparation particulièrement avantageux selon l'invention est un procédé de préparation de séquences nucléotidiques tel que défini ci-dessus, caractérisé en ce que le couple d'amorces utilisé est le suivant : (AAR GTN ATH TTY GGN AGR GA; CTC CTC TCC TCT CCT CTT CCT), R, H, N et Y étant tels que définis ci-dessus,
ledit procédé étant caractérisé en ce que :
- la première étape du procédé est une étape de dénaturation de 4 minutes à une température égale à 95°C,
- le cycle, répété 35 fois, comprend les étapes suivantes :
   * une étape de dénaturation de 30 secondes à une température égale à 95°C,
   * une étape d'hybridation de 40 secondes à une température égale à 52°C,
   * une étape d'élongation de 30 secondes à une température égale à 72°C, et
- la dernière étape du procédé est une étape d'élongation de 10 minutes à une température égale à 72°C,
afin d'obtenir une séquence nucléotidique partielle de référence pour continuer la détermination complète de cette séquence codante,
ledit procédé comprenant une étape supplémentaire de 5' RACE PCR pour obtenir la séquence nucléotidique SEQ ID NO : 9.

La séquence nucléotidique SEQ ID NO : 9 (du codon initiateur au codon Stop, c'est à dire la séquence transcrite et traduite qui correspond à un monomère de globine fonctionnel) est une nouvelle séquence nucléotidique codant pour une chaîne protéique correspondant à une chaîne de globine, notée B1. SEQ ID NO : 9 comprend 498 nucléotides.

Un procédé de préparation particulièrement avantageux selon l'invention est un procédé de préparation de séquences nucléotidiques tel que défini ci-dessus, caractérisé en ce que le couple d'amorces utilisé est le suivant : (GAR CAY CAR TGY GGN GGN GA, CTC CTC TCC TCT CCT CTT CCT), R, N et Y étant tels que définis ci-dessus,
ledit procédé étant caractérisé en ce que :
- la première étape du procédé est une étape de dénaturation de 4 minutes à une température égale à 95°C,
- le cycle, répété 35 fois, comprend les étapes suivantes :
   * une étape de dénaturation de 40 secondes à une température égale à 95°C,
   * une étape d'hybridation de 1 minute à une température égale à 58°C,
      * une étape d'élongation de 1 minute et 10 secondes à une température égale à 72°C, et
- la dernière étape du procédé est une étape d'élongation de 10 minutes à une température égale à 72°C,
afin d'obtenir une séquence nucléotidique partielle de référence pour continuer la détermination complète de cette séquence codante,
ledit procédé comprenant une étape supplémentaire de 5' RACE PCR pour obtenir la séquence nucléotidique SEQ ID NO : 11.

La séquence nucléotidique SEQ ID NO : 11 (du codon initiateur au codon Stop, c'est à dire la séquence transcrite et traduite qui correspond à un monomère de globine fonctionnel) est une nouvelle séquence nucléotidique codant pour une chaîne protéique correspondant à une chaîne de linker, notée L1. SEQ ID NO : 11 comprend 771 nucléotides.

La présente invention concerne également des séquences protéiques codées par l'une des séquences nucléotidiques telles qu'obtenues selon le procédé tel que défini ci-dessus.

Une protéine préférée selon l'invention est une protéine telle que définie ci-dessus, caractérisée en ce qu'elle comprend ou est constituée par :
- la séquence SEQ ID NO : 2 ou SEQ ID NO : 14,
- ou tout fragment d'une des séquences définies ci-dessus, sous réserve que ledit fragment permette le transport de l'oxygène, notamment tout fragment étant constitué d'au moins 60 acides aminés, et notamment d'au moins environ 160 acides aminés contigus dans la séquence SEQ ID NO : 2.

La séquence SEQ ID NO : 2 est une nouvelle séquence protéique correspondant à une chaîne entière de globine, notée A2a.

La séquence SEQ ID NO : 14 est une nouvelle séquence protéique correspondant à un fragment d'une séquence dérivée de la chaîne de globine, notée A2a, représentée par la séquence SEQ ID NO : 2.

Les propriétés de transport de l'oxygène des séquences protéiques de l'invention peuvent être notamment vérifiées en mesurant leur spectre d'absorption par spectrophotométrie typique de l'oxyhémoglobine.

Une protéine préférée selon l'invention est une protéine telle que définie ci-dessus, caractérisée en ce qu'elle comprend ou est constituée par :
- la séquence SEQ ID NO : 4 ou SEQ ID NO : 16,
- ou tout fragment d'une des séquences définies ci-dessus, sous réserve que ledit fragment permette le transport de l'oxygène, notamment tout fragment étant constitué d'au moins 60 acides aminés, et notamment d'au moins environ 160 acides aminés contigus dans la séquence SEQ ID NO : 4.

La séquence SEQ ID NO : 4 est une nouvelle séquence protéique correspondant à une chaîne entière de globine, notée A2b.

La séquence SEQ ID NO : 16 est une nouvelle séquence protéique correspondant à un fragment d'une séquence dérivée de la chaîne de globine, notée A2b, représentée par la séquence SEQ ID NO : 4.

Une protéine préférée selon l'invention est une protéine telle que définie ci-dessus, caractérisée en ce qu'elle comprend ou est constituée par :
- la séquence SEQ ID NO : 6 ou SEQ ID NO : 18,
- ou tout fragment d'une des séquences définies ci-dessus, sous réserve que ledit fragment permette le transport de l'oxygène, notamment tout fragment. étant constitué d'au moins 60 acides aminés, et notamment d'au moins environ 160 acides aminés contigus dans la séquence SEQ ID NO : 6.

La séquence SEQ ID NO : 6 est une nouvelle séquence protéique correspondant à une chaîne entière de globine, notée A1.

La séquence SEQ ID NO 18 est une nouvelle séquence protéique correspondant à un fragment d'une séquence dérivée de la chaîne de globine, notée A1, représentée par la séquence SEQ ID NO : 6.

Une protéine préférée selon l'invention est une protéine telle que définie ci-dessus, caractérisée en ce qu'elle comprend ou est constituée par :
- la séquence SEQ ID NO : 8 ou SEQ ID NO : 20,
- ou tout fragment d'une des séquences définies ci-dessus, sous réserve que ledit fragment permette le transport de l'oxygène, notamment tout fragment étant constitué d'au moins 60 acides aminés, et notamment d'au moins environ 160 acides aminés contigus dans la séquence SEQ ID NO : 8.

La séquence SEQ ID NO : 8 est une nouvelle séquence protéique correspondant à une chaîne entière de globine, notée B2.

La séquence SEQ ID NO : 20 est une nouvelle séquence protéique correspondant à un fragment d'une séquence dérivée de la chaîne de globine, notée B2, représentée par la séquence SEQ ID NO 8.

Une protéine préférée selon l'invention est une protéine telle que définie ci-dessus, caractérisée en ce qu'elle comprend ou est constituée par :
- la séquence SEQ ID NO : 10,
- ou tout fragment d'une des séquences définies ci-dessus, sous réserve que ledit fragment permette le transport de l'oxygène, notamment tout fragment étant constitué d'au moins 60 acides aminés, et notamment d'au moins environ 160 acides aminés contigus dans la séquence SEQ ID NO : 10.

La séquence SEQ ID NO : 10 est une nouvelle séquence protéique correspondant. à une chaîne de globine, notée B1.

Une protéine préférée selon l'invention est une protéine telle que définie ci-dessus, caractérisée en ce qu'elle comprend ou est constituée par :
- la séquence SEQ ID NO : 12,
- ou tout fragment d'une des séquences définies ci-dessus, sous réserve que ledit fragment permette l'association des chaînes de globines entre elles, notamment tout fragment étant constitué d'au moins 60 acides aminés, et notamment d'au moins environ 280 acides aminés contigus dans la séquence SEQ ID NO : 12.

La séquence SEQ ID NO : 12 est une nouvelle séquence protéique correspondant à une chaîne de Linker, notée L1.

La présente invention concerne également des séquences nucléotidiques telles qu'obtenues selon le procédé tel que défini ci-dessus.

La présente invention concerne également des séquences nucléotidiques codant pour une protéine telle que définie ci-dessus.

La présente invention concerne également une séquence nucléotidique telle que définie ci-dessus, caractérisée encre qu'elle comprend ou est constituée par :
- la séquence nucléotidique SEQ ID NO: 1 ou SEQ ID NO:13 codant respectivement pour SEQ ID NO : 2 ou SEQ ID NO: 14,
- ou toute séquence nucléotidique dérivée, par dégénérescence du code génétique, de la séquence SEQ ID NO 1 ou SEQ ID NO : 13, et codant respectivement pour une protéine représentée par SEQ ID NO : 2 ou SEQ ID NO : 14,
- ou tout fragment de la séquence nucléotidique SEQ ID NO : 1 ou des séquences, nucléotidiques définies ci-dessus, ledit fragment étant de préférence constitué d'au moins environ 180 nucléotides, et notamment d'au moins environ 480 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

Les conditions de stringence correspondent à des gammes de températures comprises entre 48 et 60°C et de concentrations en MgCl₂ comprises entre 1 et 3 mM.

La présente invention concerne également une séquence nucléotidique telle que définie ci-dessus, caractérisée en ce qu'elle comprend ou est constituée par :
- la séquence nucléotidique SEQ ID NO : 3 ou SEQ ID NO : 15 codant respectivement pour SEQ ID NO: 4 ou SEQ ID NO : 16,
- ou toute séquence nucléotidique dérivée, par dégénérescence du code génétique, de la séquence SEQ ID NO : 3 ou SEQ ID NO : 15, et codant respectivement pour une protéine représentée par SEQ ID NO: 4 ou SEQ ID NO : 16,
- ou tout fragment de la séquence nucléotidique SEQ ID NO : 3 ou SEQ ID NO : 15 ou des séquences nucléotidiques définies ci-dessus, ledit fragment étant de préférence constitué d'au moins environ 180 nucléotides, et notamment d'au moins environ 480 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

La présente invention concerne également une séquence nucléotidique telle que définie ci-dessus, caractérisée en ce qu'elle comprend ou est constituée par :
- la séquence nucléotidique SEQ ID NO: 5 ou SEQ ID NO : 17 codant respectivement pour SEQ ID NO : 6 ou SEQ ID NO : 18,
- ou toute séquence nucléotidique dérivée, par dégénérescence du code génétique, de la séquence SEQ ID NO : 5 ou SEQ ID NO : 17; et codant respectivement pour une protéine représentée par SEQ ID NO : 6 ou SEQ ID NO : 18,
- ou tout fragment de la séquence nucléotidique SEQ ID NO : 5 ou SEQ ID NO : 17 ou des séquences nucléotidiques définies ci-dessus, ledit fragment étant de préférence constitué d'au moins environ 180 nucléotides et notamment d'au moins environ 480 nucléotides contigus dans ladite séquence,
- ou toute séquence nucléotidique complémentaire des séquences ou fragments susmentionnés,
- ou toute séquence nucléotidique: susceptible d'hybrider dans des conditions stringentes avec la séquence complémentaire d'une des séquences ou d'un des fragments susmentionnés.

La présente invention concerne également une séquence nucleotidique telle que définie ci-dessus, caractérisée en ce qu'elle comprend ou est constituée par :
- la séquence nucléotidique SEQ ID NO : 7 ou SEQ ID NO : 19 codant respectivement pour SEQ ID NO : 8 ou SEQ ID NO : 20,
- ou toute séquence: nucléotidique dérivée, par dégénérescence du code génétique, de la séquence SEQ ID NO : 7 ou SEQ ID NO : 19, et codant respectivement pour une protéine représentée par SEQ ID NO : 8 ou SEQ ID NO : 20,
- ou tout fragment de la séquence nucléotidique SEQ ID NO : 7 où SEQ ID NO : 19 ou des séquences nucléotidiques définies ci-dessus, ledit fragment étant de préférence constitué d'au moins environ 180 nucléotides, et notamment d'au moins environ 480 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.
   La présente invention concerne également une séquence nucléotidique telle que définie ci-dessus, caractérisée en ce qu'elle comprend ou est constituée par :
- la séquence nucléotidique SEQ ID NO : 9 codant pour SEQ ID NO : 10,
- ou toute séquence nucléotidique dérivée, par dégénérescence du code génétique, de la séquence SEQ ID NO : 9, et codant pour une protéine représentée par SEQ ID NO : 10,
- ou tour fragment de la séquence nucléotidique SEQ ID NO: 9 ou des séquences nucléotidiques définies ci-dessus, ledit fragment étant de préférence constitué d'au moins environ 180 nucléotides, et notamment d'au moins environ 480 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.
   La présente invention concerne également une séquence nucléotidique telle que définie ci-dessus, caractérisée en ce qu'elle comprend ou est constituée par
- la séquence nucléotidique SEQ ID NO : 11 codant pour SEQ ID NO : 12,
- ou tout fragment de la séquence nucléotidique SEQ ID NO : 11 ou des séquences nucléotidiques définies ci-dessus, ledit fragment étant de préférence constitué d'au moins environ 180 nucléotides, et notamment d'au moins environ 800 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

La présente invention concerne un procédé de préparation tel que défini ci-dessus, de séquences nucléotidiques codant pour les chaînes protéiques constituant la molécule d'hémoglobine d'Annélides, notamment d'Arenicola marina, ledit procédé étant caractérisé en ce qu'il comprend les étapes suivantes :
- une étape de mise en présence de la molécule d'hémoglobine susmentionnée avec au moins un agent dissociant et un agent réducteur, notamment un mélange constitué de dithiothréitol (DTT) ou de chlorhydrate de tris(2-carboxyéthyl)phosphine (TCEP) ou de bêta-mercaptoéthanol et d'un tampon de dissociation, pendant un temps suffisant pour séparer les chaînes protéiques les unes des autres,
   permettant la dissociation, puis la réduction de ladite molécule d'hémoglobine, afin d'obtenir les chaînes protéiques constituant ladite molécule,
- l'isolement des chaînes protéiques susmentionnées,
- le microséquençage par spectrométrie de masse et le séquençage d'Edman de chacune des chaînes protéiques isolées susmentionnées, afin d'obtenir une microséquence correspondant à chacune des séquences constituées de 5 à 20 acides aminés,
- la détermination des couples d'amorces dégénérées (sens et antisens) à partir des micro séquences susmentionnées,
- la préparation des séquences nucléotidiques codant pour les chaînes protéiques susmentionnées, à partir des amorces telles qu'obtenues précédemment, par un procédé d'amplification en chaîne par polymérase (PCR), comprenant les étapes suivantes :
- la première étape dudit procédé est une étape de dénaturation de l'ADNc codant pour les chaînes protéiques constituant la molécule d'hémoglobine *d'Arenicola marina,* d'environ 10 secondes à environ 5 minutes à une température comprise d'environ 90°C à environ 110°C,
- le cycle, répété environ 30 à 40 fois, comprend les étapes suivantes:
   * une étape de dénaturation de l'ADNc codant pour les chaînes protéiques constituant la molécule d'hémoglobine d'*Arenicola marina,* d'environ 10 secondes à environ 5 minutes, à une température comprise d'environ 90°C à environ 110°C,
   * une étape d'hybridation des couples d'amorces de l'invention aux brins d'ADNc monocaténaires susmentionnés pour obtenir des amorces hybridées, d'environ 20 secondes à environ 2 minutes, à une température comprise d'environ 50°C à environ 56°C,
   * une étape d'élongation des amorces hybridées telles qu'obtenues précédemment par une polymérase d'environ 20 secondes à environ 1 minute et 30 secondes, à une température comprise d'environ 70°C à environ 75°C, et
- la dernière étape du procédé est une étape d'élongation des amorces hybridées telles qu'obtenues précédemment par une polymérase d'environ 5 minutes à environ 15 minutes à une température comprise d'environ 70°C à environ 75°C.

### DESCRIPTION DES FIGURES

La Figure 1 représente un chromatogramme de l'hémoglobine de *Arenicola marina* sur colonne Superose 12-C. La courbe supérieure correspond à une absorbance de 414 nm et la courbe inférieure à une absorbance de 280 nm. (Le collecteur est programmé pour collecter entre 16 et 18 minutes).
La Figure 2 représente le spectre UV de l'hémoglobine de *Arenicola marina* fonctionnelle (sous sa forme oxyhémoglobine).
La Figure 3 représente le chromatogramme (à 414 nm) de l'HbAm dissociée (partiellement) obtenu sur Superose 12-C et les traits verticaux sur le chromatogramme correspondent aux fenêtres de collecte (correspondant à la récupération des sous-unités).
La Figure 4 représente un gel SDS-PAGE obtenu pour les différentes fractions collectées.
La Figure 5 représente le chromatogramme (à 414 nm) de l'HbAm dissociée (partiellement) obtenu sur CIM DISK DEAE (système d'échange anionique) et les traits verticaux sur le chromatogramme correspondent aux fenêtres de collecte. La courbe en pointillés indique le gradient.
La Figure 6 représente un gel SDS-PAGE obtenu pour les différentes fractions collectées.
La Figure 7 représente la cinétique de dissociation de l'HbAm en présence d'urée 3M. L'axe des abscisses correspond au nombre de jours et l'axe des ordonnées correspond au pourcentage de dissociation de la molécule native ; la courbe en pointillés correspond au dodécamère ; la courbe avec les carrés noirs au trimère et au "linker" (chaîne de structure) ; la courbe avec les ronds noirs aux monomères.
La Figure 8 représente la cinétique de dissociation de l'HbAm à pH 10. L'axe des abscisses correspond au nombre de jours et l'axe des ordonnées correspond au pourcentage de dissociation de la molécule native ; la courbe en pointillés correspond au dodécamère ; la courbe avec les carrés noirs au trimère et au "clinker" ; la courbe avec les ronds noirs aux monomères.
La Figure 9 représente la cinétique de dissociation de l'HbAm en présence d'urée 3M à pH 10. L'axe des abscisses correspond au nombre de jours et l'axe des ordonnées correspond au pourcentage de dissociation de la molécule native ; la courbe en pointillés correspond au dodécamère ; la courbe avec les carrés noirs au trimère et au "linker" ; la courbe avec les ronds noirs aux monomères.

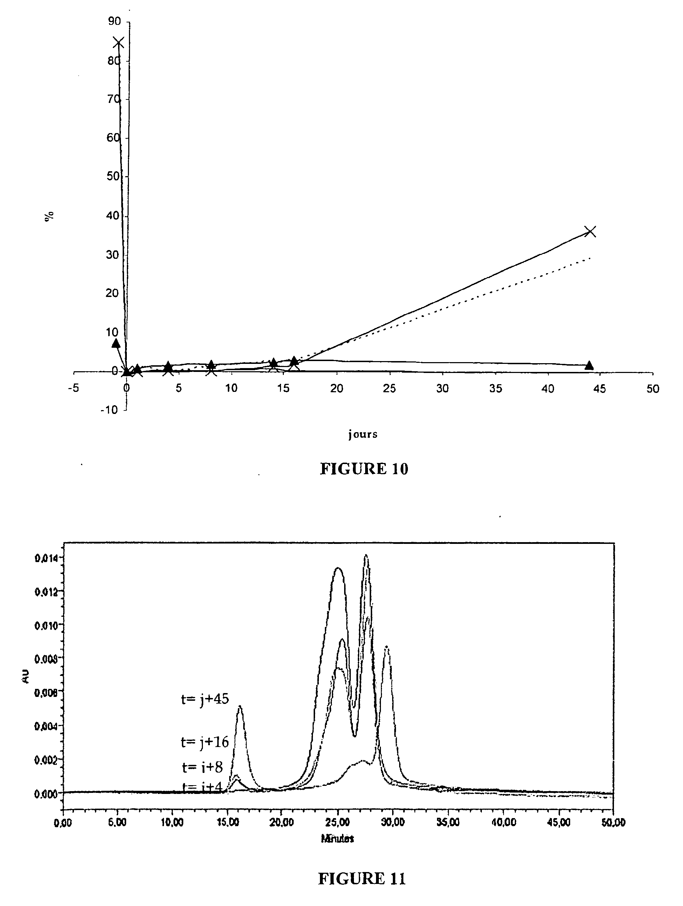

La Figure 10 représente le suivi de la cinétique de réassociation à partir du pourcentage d'HbAm (HBL) et de Dodécamère (D) et selon la technique de changement de tampon (Centricon ou Dialyse). L'axe des abscisses correspond au nombre de jours et l'axe des ordonnées correspond au pourcentage de dissociation de la molécule native avec la technique Centricon ; la courbe en traits pointillés correspond à HBL avec la technique de dialyse ; la courbe avec les triangles noirs correspond au dodécamère avec la technique Centricon ; la courbe en trait plein correspond au dodécamère avec la technique de dialyse.
La Figure 11 représente la superposition des chromatogrammes de chromatographie d'exclusion au cours de la réassociation correspondant à différents temps de réassociation.
La Figure 12 représente le chromatogramme HPLC obtenu après séparation des chaînes polypeptidiques par phase inverse sur une colonne Symmetry C18 (Waters). Les codes (d2, a1, a2, b2, c) correspondent aux noms des globines telles que mentionnées dans l'article de Zal et al. (1997).

### PARTIE EXPÉRIMENTALE

La présente invention a pour objectif l'utilisation de l'hémoglobine extracellulaire du polychaete marin *Arenicola marina* (HbAm) comme substitut sanguin chez des vertébrés. Cependant, même si ce ver représente une biomasse importante, la synthèse par génie génétique s'avère une voie indispensable et nécessaire. Il est donc primordial d'obtenir les séquences primaires des chaînes protéique constituant HbAm afin de développer une hémoglobine artificielle, fonctionnelle et stable à partir des propriétés d'auto-assemblage de cette molécule. Les protocoles de dissociations de chaque sous-unité et de réduction en chaîne polypeptidique, ainsi que les techniques de purification, d'isolement de microséquençage et de séquençage de ces chaînes et sont développées en détail ci-après.

### Extraction et purification de l'hémoglobine

### 1) Espèce étudiée : Arenicola marina ; Annélide de l'écosystème intertidal

L'Annélide Polychète *Arenicola marina* est une espèce sédentaire très répandue sur toutes les côtes de l'Atlantique Nord, de la mer Noire et de l'Adriatique situées au-dessus du quarantième parallèle. Dans la région de Roscoff, l'Arénicole, communément appelée "ver du pêcheur" constitue des populations denses. Le sédiment habité par ces populations présente une surface irrégulière alternant bosses et creux formés respectivement par des monticules de déjections et des dépressions coniques. L'Arénicole vit dans des galeries aménagées dans le sable. La structure de la galerie se présente sous forme de U, avec une branche ouverte sur l'extérieur, l'autre étant fermée. L'Arénicole est logée dans la partie horizontale, son extrémité céphalique orientée vers la partie aveugle. Elle ingère le sable, en extrait la matière organique assimilable et défèque ensuite par son extrémité caudale, formant ainsi des monticules de tortillons de sable. A l'intérieur de l'étage médiolittoral, la répartition et la densité des peuplements sont essentiellement commandées par la granulométrie, la concentration en matière organique et la salinité.

L'Arénicole, vivant surtout dans les zones de balancement des marées, est amenée à subir des variations de pression d'oxygène. Sa galerie lui permet d'être en contact permanent avec l'eau de mer (riche en oxygène), pendant la marée basse.

### 2) Méthodes d'étude

### 2.1. Prélèvement du matériel biologique

Les animaux sont récoltés à marée basse, dans la baie de Penpoull, près de Roscoff (France) et maintenus dans l'eau de mer une nuit afin de vider leur tube digestif. Les prélèvements de sang sont effectués à l'aide d'une seringue au niveau du vaisseau dorsal. Les échantillons sont collectés sur de la glace et filtrés sur de la laine de verre. Après une centrifugation à froid (15 000 g pendant 15 min à 4°C) pour éviter la dissociation des molécules et pour éliminer les débris tissulaires, les surnageants sont concentrés au moyen d'une cellule Amicon (Millipore) et d'une membrane de "cut-off" de 500 kDa (ne sont retenues que les masses supérieures à 500 kDa).

### 2.2. Purification des hémoglobines

Une fois le sang concentré, une filtration basse pression (FPLC) par exclusion (séparation en fonction de la taille de la molécule : plus la molécule est de taille importante plus elle est éluée rapidement) est effectuée sur colonne (100 × 3 cm) de gel Sephacryl S-400 (Amersham)(gamme de séparation comprise entre 20 × 10³ et 8000 × 10³), en chambre froide (4°C). Chaque purification est effectuée sur 5 mL d'échantillon, élués avec le tampon salé *Arenicola marina* (10 mM Hepes ; 4 mM KCl ; 145 mM NaCl ; 0,2 mM MgCl₂ ajusté à pH 7,0 avec de la soude 2N). Le débit utilisé pour cette première purification est de 40 tr/min et n'est récupéré que la première fraction la plus rouge (contenant de l'hème). Cette fraction est ensuite concentrée à l'aide de tube Centricon-10kDa retenant les molécules d'un poids supérieur ou égal à 10000 Da.

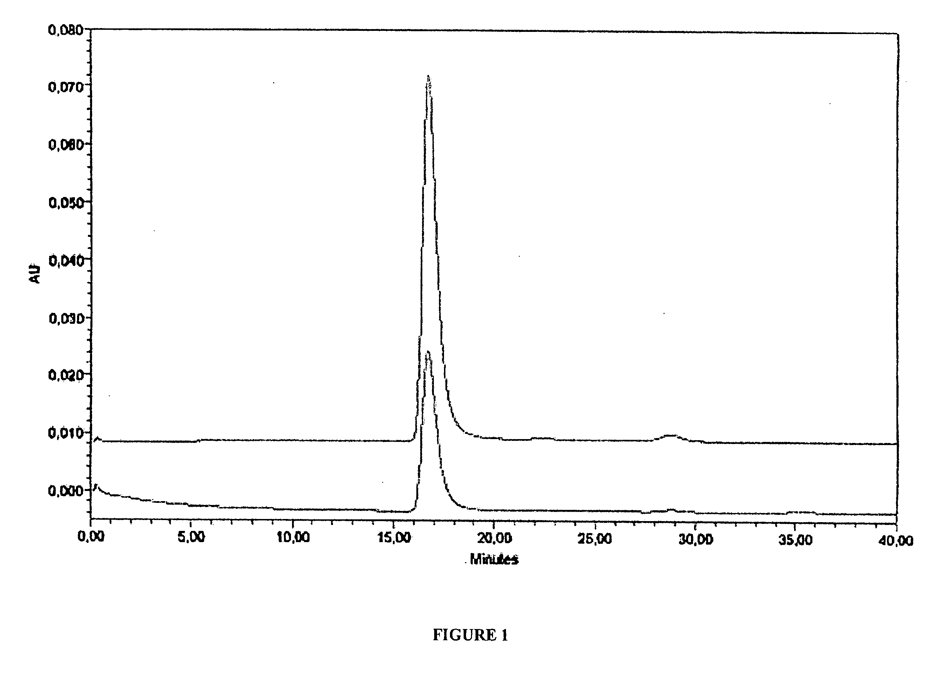

Une deuxième purification est ensuite effectuée par filtration basse pression (Système HPLC, Waters) d'aliquot de 200 µL sur une colonne 1 × 30 cm Superose 12-C (Pharmacia, gamme de séparation comprise entre 5 × 10³ et 3 × 10⁵ Da) à température ambiante. Le débit utilisé est de 0,5 ml/min. Les échantillons sont conservés à 4°C et récoltés dans de la glace. L'absorbance de l'éluat est suivie à deux longueurs d'ondes : 280 nm (pic d'absorbance caractéristique des protéines) et 414 nm (pic d'absorbance caractéristique de l'hème). Les fractions contenant de l'hème sont isolées grâce au collecteur (programmé sur une fenêtre de temps correspondant au temps de rétention de l'hémoglobine)(Figure 1). Les échantillons sont concentrés, dosés, puis stockés à -40°C avant utilisation.

### 2.3. Dosage des hémoglobines

Le réactif de Drabkin (Sigma), utilisé pour le dosage, permet de déterminer la quantité en hème de la solution. L'hémoglobine réagit avec le réactif de Drabkin lequel contient du potassium de ferricyanure, du cyanure de potassium et du bicarbonate de sodium. L'hémoglobine est transformée en methémoglobine par l'action du ferricyanure. Les methémoglobines réagissent ensuite avec le cyanure pour former le cyanmethémoglobine. L'absorbance de ce dérivé à 540 nm est proportionnelle à la quantité d'hème dans la solution. Or, l'hémoglobine extracellulaire de *Arenicola marina* (HBL) contient en moyenne 1 mol d'hème pour 23 000 g de protéine, ce qui permet par un calcul simple, d'obtenir la concentration en HBL de chaque échantillon.

### 3) Résultats

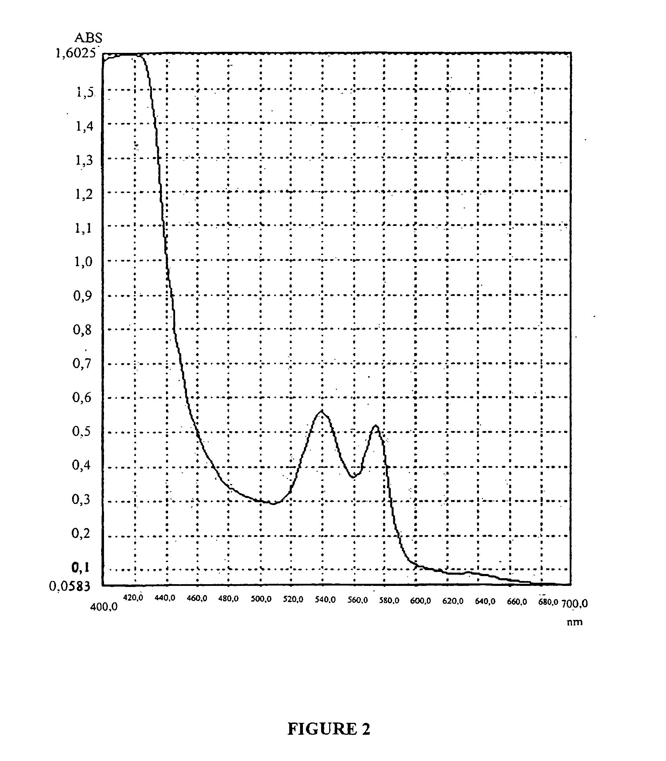

Ainsi, plusieurs milligrammes d'hémoglobine extracellulaire de *Arenicola* marina ont été purifiés. Chaque lot (aliquots de 1mL) est analysé par FPLC sur colonne Superose 12-C (Pharmacia) afin de s'assurer de la pureté de l'échantillon (un pic unique). De même un spectre UV sur une plage de 400 nm à 700 nm est réalisé pour vérifier la fonctionnalité des hémoglobines de chaque lot (Figure 2). Trois maxima d'absorption sont observés à 414, 541 et 577 nm. Par comparaison, on rappelle que la méthémoglobine présente deux maxima à 500 et 635 nm.

Enfin, ces lots sont utilisés par la société Biotrial S.A. (Rennes, France) pour des tests précliniques réalisés sur des souris et des rats afin de tester les éventuelles réactions pathologiques et immunogènes.

### Dissociation de l'hémoglobine extracellulaire de Arenicola marina en ces différentes sous-unités de base (Trimères, Dimères de linker et Monomères)

La dissociation de l'hémoglobine extracellulaire d'*Arenicola marina* (HbAm) doit être totale et conserver les sous-unités fonctionnelles. Les différentes sous-unités sont ensuite isolées et analysées par des techniques de chromatographie liquide (exclusion et échange d'ions), développées à cet effet.

### 1) Dissociation de l'HBL

### 1.1. Protocole de dissociation

Les études préliminaires de dissociation ont été développées à partir des publications de l'art antérieur (Vinogradov et al., 1979 ; Sharma et al., 1996 ; Mainwaring et al., 1986 ; Polidori et al., 1984 ; Kapp et al., 1984 ; Chiancone et al., 1972 ; Vinogradov et al., 1991 ; Krebs et al., 1996), c'est-à-dire en présence d'un réactif dissociant unique : urée, ions hétéropolytungstate, sels de guanidinium, SDS ou ions hydroxydes. Les agents utilisés agissent différemment sur la molécule :
- les ions hydroxydes (OH-), le SDS, les sels de guanidinium et les ions hétéropolytungstate déstabilisent les ponts salins
- l'urée déstabilise les interactions hydrophobes

Or, l'intérêt est d'obtenir le plus rapidement et le plus efficacement possible les quatre sous-unités de bases d'où l'idée d'associer les différents agents dissociants et en particulier le pH alcalin et l'urée. Après différents tests, il s'avère qu'une dissociation rapide et efficace est obtenue avec de l'urée 3M dilué dans le tampon de dissociation (0,1 M de base Trisma et 1mM d'EDTA) ajusté à pH 10 avec de la soude 2N. L'HbAm est ajustée à une concentration d'environ 4 mg/mL (solution mère). Toutes les analyses sont effectuées à +4°C et les échantillons sont conservés à l'obscurité le temps de l'étude. (Trisma = tris[hydroxyméthyl]aminométhane)

### 1.2. Analyses par chromatographie d'exclusion

Les conditions d'analyses sont détaillées dans le tableau 1 ci-dessous.

| Système HPLC | HPLC Waters 626 LC System | |
|---|---|---|
| Colonne | Superose 12-C (Pharmacia) (gamme de séparation comprise entre 5x10³ et 3x10⁵ Da) | |
| Débit | 0.5 mL/min | |
| Eluant | tampon pH 7,0 (*tamponné par exemple avec de l'acide chlorhydrique concentré)* et filtré sur filtre 0,22 µm (ou 0,45µm) | |
| Température de l'injecteur | +4°C | |

| | Echantillon | |
|---|---|---|
| | Suivi Analytique | Séparation et collecte |
| Volume injecté | 20µL | 200µL |
| Préparation des échantillons | Solution mère diluée à 1mg/mL dans le tampon de dissociation à pH10 et filtrée sur 0,45gm | Solution mère filtrée sur filtre 0,45µm |

### 1.3. Analyses par échange d'ions

Le point isoélectrique (pHi) de l'HBL étant de 4,69 (Vinogradov, 1985), l'analyse par échange d'ions est réalisée sur une colonne anionique CIM DEAE disk (Interchim). En effet, L'HBL est chargée négativement pour un pH supérieur au pHi et est donc fixée sur une résine chargée positivement (résine DEAE). L'élution est effectuée grâce à la force ionique avec un gradient non linéaire de NaCl de 0 à 1 M (solution de NaCl de 1 M diluée dans le tampon de dissociation à pH 7,0 et filtrée sur 0,45 µm). Le tampon de dissociation à pH 7 est utilisé comme tampon d'élution. Le débit est de 4 mL/min.

### 1.4. Analyses par chromatographie en phase inverse

La chromatographie par phase inverse est effectuée sur une colonne de Symmetry 300 C₁₈ 5µm (4,6 × 250 mm) de chez Waters. En présence d'acétonitrile et de TFA (acide trifluoroacétique), HbAm se dissocie en ses sous unités de base (Trimère, Monomère et Linker) et l'hème se dissocie des globines. Ainsi, sans traitement préalable, HbAm est dissociée en tête de colonne. La méthode développée est décrite dans le tableau ci-dessous.

| | | | |
|---|---|---|---|
| débit | 1 mL/min | | |
| Eluant A | H₂O MilliQ + 0.1 % v/v HFBA | | |
| Eluant B | ACN + 0.1 % v/v HFB A | | |

| Gradient | | | |
|---|---|---|---|
| | Temps en min | %A | %B |
| | 0 | 58 | 42 |
| | 40 | 50 | 50 |
| | 41 | 48 | 52 |
| | 90 | 47 | 53 |
| | 95 | 5 | 95 |
| | 110 | 5 | 95 |

### 2) Résultats

### 2.1. Chromatographie d'exclusion

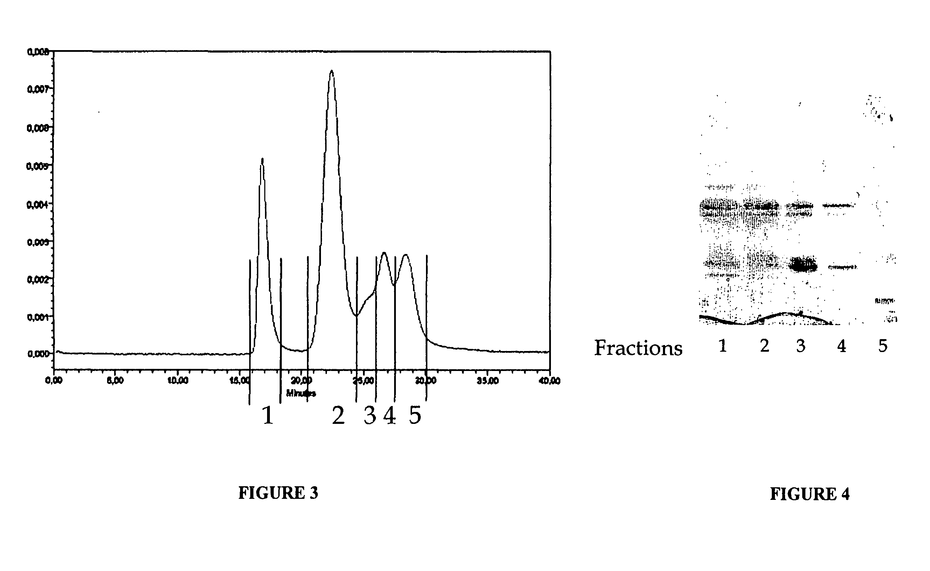

Le chromatogramme de l'HbAm dissociée en partie, est représenté figure 3. Cinq pics sont observés et il s'agit de les identifier. Les molécules sont éluées selon leur masse décroissante et l'HbAm native élue dans le volume mort (16 min). Les fractions correspondant à chaque pic sont analysées par SDS-PAGE (figure 4). Les résultats sont présentés dans le tableau 2 ci-dessous.

| **Fractions** | **Temps de rétention** | **Sous-unités** |
|---|---|---|
| 1 | 16 min 40 | HbAm native |
| 2 | 22 min 20 | Dodécamère |
| 3 | 25 min 30 | Dimère de linker |
| 4 | 26 min 40 | Trimère |
| 5 | 28 min 30 | Monomères |

### 2.2. Chromatographie ionique

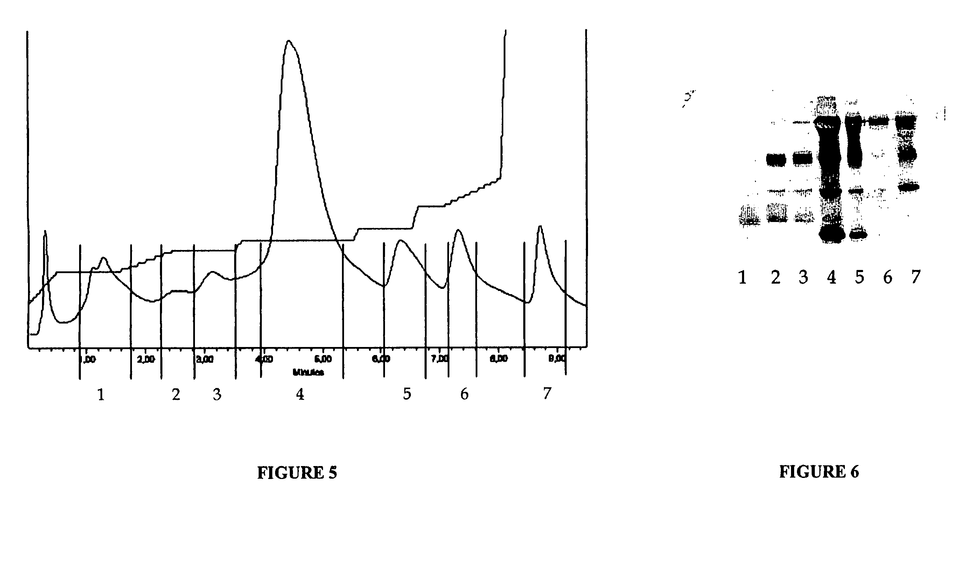

Une fois la méthode développée (tableau 3), les fractions sont collectées, concentrées et analysées par gel SDS afin d'identifier chaque pic (Fig. 6 et tableau 4).

Une méthode est ensuite développée pour repurifier chaque sous-unité.

**TABLEAU 3 : Méthode développée pour l'analyse de l'HBL dissociée sur CIM-DEAE**

| **Temps en min** | **A : 1M NaCl dans B** | **B : Tampon de dissociation pH 7.0** |
|---|---|---|
| 0 | 5% | 95 % |
| 0,5 | 15% | 85 % |
| 1,5 | 15% | 85% |
| 2,5 | 22% | 78% |
| 3,5 | 22% | 78% |
| 3,6 | 25% | 75% |
| 5,5 | 25% | 75% |
| 5,6 | 29% | 71% |
| 6,5 | 29% | 71 % |
| 6,6 | 36% | 64% |
| 7,0 | 36% | 64% |
| 8,0 | 45% | 55% |
| 8,1 | 100% | 0% |
| 9,5 | 100% | 0% |

**TABLEAU 4: Association réalisée après analyse du gel (Figure 6) entre le temps de rétention et les sous-unités.**

| **fractions** | **Temps de rétention** | **Sous-unités** |
|---|---|---|
| 1 | 1 min 15 | Monomères |
| 2 | 2 min 40 | Dimère de linker |
| 3 | 3 min 10 | Dimère de linker |
| 4 | 4 min 40 | Dodécamère |
| 5 | 6 min 30 | ? |
| 6 | 7 min 30 | Trimère |
| 7 | 8 min 50 | HbAm |

### 2.3. Chromatographie en phase inverse

Une fois la méthode développée, les fractions sont collectées, lyophilisées et analysées par spectrométrie de masse afin d'identifier chaque pic.

| fractions | Temps de rétention | Sous-unités |
|---|---|---|
| 1 | 12 min | Linker |
| 2 | 22 min | Linker |
| 3 | 34 min | Monomère a1 |
| 4 | 38 min | Monomère a2 |
| 5 | 50 -70 min | Trimères |

Les propriétés chimiques des trimères doivent être trop proches pour que l'on puisse les séparer par phase inverse. Ainsi, seuls les deux linkers et les monomères a1 et a2 ont pu être isolés.

### 2.4. Dissociation de l'HbAm

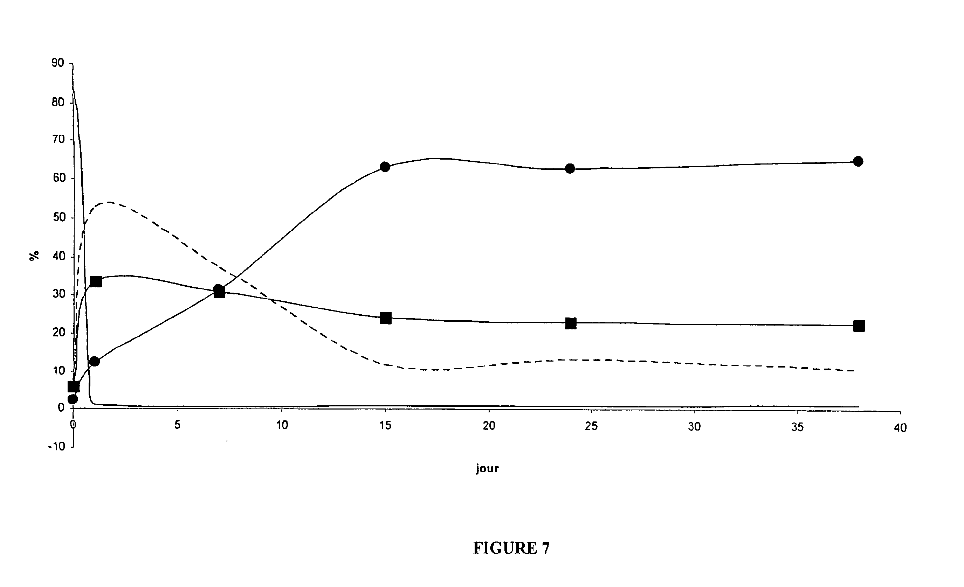

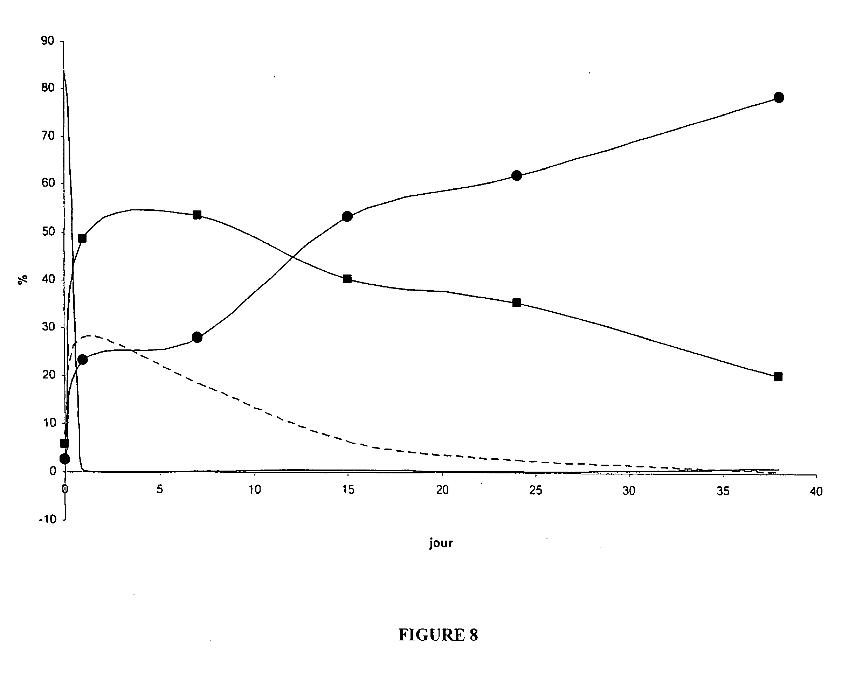

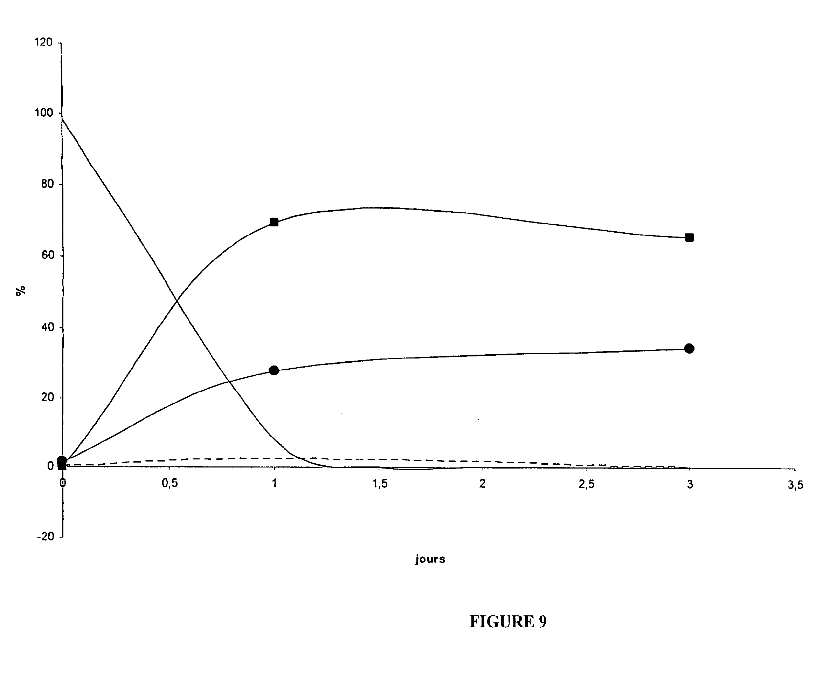

La cinétique de dissociation est suivie par chromatographie d'exclusion. L'intégration des chromatogrammes par le logiciel Millénium (Waters), permet le calcul du pourcentage des différents composés à partir de l'aire sous courbe. L'évolution de la cinétique de dissociation est représentée figures 7, 8 et 9.

Les trois graphiques des Figures 7, 8 et 9 montrent bien l'intérêt d'associer les deux agents dissociants (urée 3M et OH⁻) pour obtenir efficacement les trois sous-unités de base en 24h.

### Réassociation de l'hémoglobine

### 1) Matériels et méthodes

Les expériences de réassociation sont effectuées sur l'HbAm dissociée selon les protocoles cités plus haut (pH9, pH10, Urée 3M, Urée 4M, Urée 3M à pH10). Différents tampons de réassociation sont testés afin d'obtenir une réassociation optimale. Le changement de tampon (tampon de dissociation → tampon de réassociation) est effectué de deux façons différentes :
- L'HbAm dissociée est lavée 4 fois contre 4 mL de tampon de réassociation sur Centricon-10 (Millipore) à +4°C ;
- L'HbAm dissociée est dialysée 24 h contre de l'eau MilliQ (Millipore) (2 × 2L) puis 48 h contre le tampon de réassociation (3 × 2L) à +4°C.

### 2) Résultats

D'après des résultats ultérieurs sur les hémoglobines extracellulaires d'Annélides (Mainwaring et al., 1986 ; Polidori et al., 1984), la présence d'ions divalents tels que Ca²⁺ et Mg²⁺ est nécessaire au maintien de la structure quaternaire de l'hémoglobine. En effet, ils la stabilisent et ralentissent le phénomène de dissociation (Sharma et al., 1996). Ces ions forment un complexe avec les groupes carboxylates des chaînes latérales et carbonyles des chaînes principales. La présence d'ions divalents peut avoir un effet sur la réassociation lorsque les groupements carboxyliques sont ionisés, donc entre autre lorsque la dissociation a eu lieu à pH alcalin. II est donc important que le tampon de réassociation contienne du calcium et/ou du magnésium. Ceci explique aussi la présence d'EDTA dans le tampon de dissociation ; EDTA qui chélate ces ions divalents. Le tampon actuellement mis au point est constitué de 0,1 M de base Trisma, 400 mM de NaCI, 2,95 mM KCl, 32 mM MgSO₄, 11 mM CaCl₂ ajusté à pH 7 avec de l'HCl concentré. La réassociation est suivie selon le même principe que la dissociation (Figures 10 et 11).

Une réassociation est observée si la dissociation est de courte durée de l'ordre de la minute. Cette réassociation correspond à un réarrangement d'intermédiaires de dissociation qui sont des hémoglobines tronquées (HBL dissociée de 1 à plusieurs douzièmes).

### Réduction de l'HbAm pour l'étude des différentes chaînes polypeptidiques

### 1) Réduction de l'hémoglobine préalable à la séparation par Chromatographie liquide par phase inverse

L'HbAm (4 mg/mL) est réduite dans 10% de DTT (dithiothréitol) dissous dans un tampon de dissociation à pH 8-9 (0,1 M trisma ou 10 mM bicarbonate d'ammonium) pendant 30 minutes à température ambiante. Une fois réduites, les chaînes protéiques obtenues sont alkylées en présence de 100mM iodoacétamide dissout dans un tampon de dissociation à pH 8-9 pendant 30 minutes à température ambiante.

On peut éventuellement également envisager le protocole suivant : L'HbAm (4 mg/mL) est réduite dans 100 mM de DTT (dithiothréitol) dissout dans le tampon de dissociation à pH 8-9 pendant 1 h à 40°C. Dans ces conditions drastiques, seules les globines pourront être analysées. En effet, les linkers (protéines non-globines) sont riches en cystéines et sont donc détériorés. Une fois réduite, l'HbAm est lavée 4 fois sur Amicon 30 000Da (Millipore) dont seul le filtrat est récupéré (tout ce qui est de poids inférieur à 30 000 Da). Le filtrat est ensuite lavé sur Amicon 10 000Da pour éliminer tout ce qui est inférieur à 10 000 Da. Ainsi, seuls les monomères compris entre 30 000 Da et 10 000 Da sont contenus dans l'échantillon (gamme de poids des chaînes de globines qui constituent l'HbAm).

### 2) Séparation des chaînes protéiques par chromatographie de phase inverse

### 2.1. Matériels et méthodes

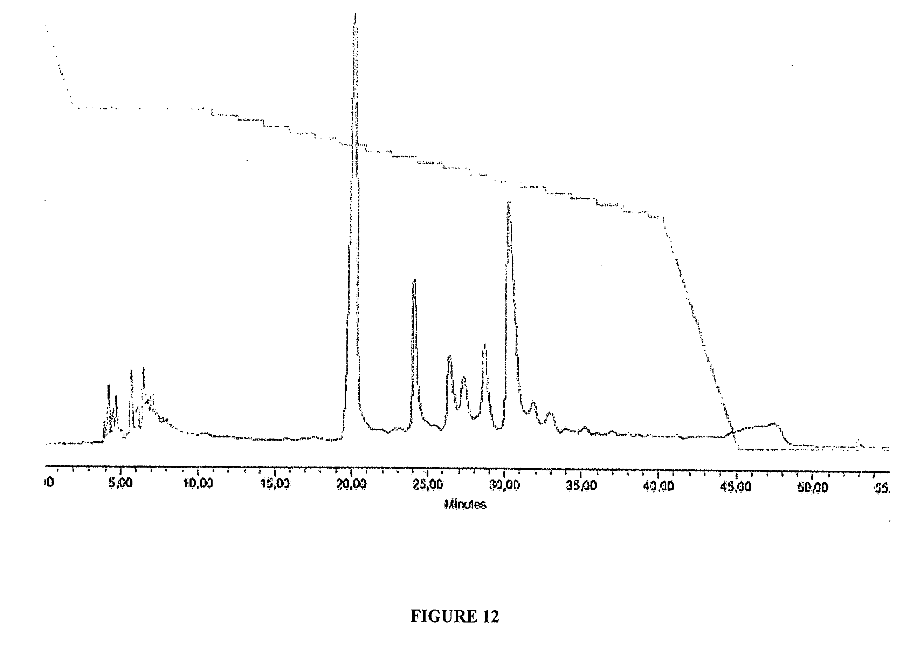

La chromatographie par phase inverse est effectuée sur une colonne de Symmetry 300 C₁₈ 5µm (4,6 × 250 mm) de chez Waters. La méthode développée est décrite dans le tableau ci-dessous et le chromatogramme obtenu est représenté figure 12.

| | | | |
|---|---|---|---|
| **débit** | 1 mL/min | | |
| **Eluant A** | H₂O MilliQ + 0.1 % v/v HFBA | | |
| **Eluant B** | ACN + 0.1 % v/v HFBA | | |

| | **Gradient** | | |
|---|---|---|---|
| | Temps en min | %A | %B |
| | 0 | 75 | 25 |
| | 2 | 58 | 42 |
| | 10 | 58 | 42 |
| | 40 | 40 | 60 |
| | 45 | 0 | 100 |
| | 55 | 0 | 100 |

On peut éventuellement également envisager le protocole suivant.

| | | |
|---|---|---|
| **débit** | 1 mL/min | |
| **Eluant A** | H₂O MilliQ + 0. 1 % v/v TFA | |
| **Eluant B** | ACN + 0.1% v/v TFA | |

| **Gradient** | | |
|---|---|---|
| Temps en min | %A | %B |
| 0 | 80 | 20 |
| 0,25 | 60 | 40 |
| 4,0 | 55 | 45 |
| 10,0 | 55 | 45 |
| 10,05 | 0 | 100 |
| 16,0 | 0 | 100 |

Chaque chaîne protéique (mises en évidence par un pic unique à 280nm) est collectée puis lyophilisée et stockée à -40°C jusqu'aux prochaines analyses. Ainsi, il a été possible de séparer les 5 monomères suivants : a₁ (~15952 kDa), a₂ (~15975 kDa), d₂ (~17033 kDa), b₂ (-16020 kDa) et c (~16664 kDa).

### 3) Séparation des chaînes protéiques par gel bidimensionnel

Le gel bidimensionnel qui est une combinaison des techniques d'isoélectrofocalisation en première dimension et de SDS-PAGE en seconde dimension permet de séparer un mélange complexe de protéine.

### Electrofocalisation

Après purification par FPLC, l'hémoglobine de *Arenicola* est dialysée et lyophilisée. 500 µg sont repris dans un tampon de réhydratation. Ce tampon contient du Chaps (3-[(3-cholamidopropyl)diméthylammonio]-1-propane-sulfonate ; Sigma) à 4%, 1% de DTT fraîchement préparé, cocktail d'inhibiteurs de protéase (Bohringher), 50 µg/ml de TLCK (inhibiteur de la trypsine), 1 µl de Bleu de Bromophénol à 1 %.

Le mélange est soniqué et centrifugé pour éliminer le matériel non dissous. On dépose ensuite de l'huile de roche aux deux extrémités du support de la bande d'électrofocalisation, et on dépose ensuite l'échantillon au milieu. La bande de 17 cm est ensuite déposée sur l'échantillon en éliminant toute bulle d'air. La bande est alors recouverte d'huile de roche pour éviter l'évaporation de l'échantillon.

Une réhydratation active est alors réalisée à 50V (20°C durant 12 heures). Ensuite nous procédons à la focalisation durant deux jours.

La bande sera ensuite récupérée et placée sur un gel d'acrylamide 6-18%, notamment à 10%, de largeur 18 cm, de longueur 20 cm et d'épaisseur 1 mm. La migration est effectuée en enceinte réfrigérée à 10°C, pendant 14 heures à 400 V, 25 mA et 100 W.

La séparation des chaînes protéiques sera alors réalisée en fonction de leur taille après avoir scellé la bande en haut du gel à l'aide d'une solution d'agarose à 1%. Une fois séparées, les bandes protéiques sont révélées sur gel par coloration au bleu de Coomassie (Coomassie^{®} G250).

### Construction du gel en gradient

Vingt-cinq ml de solution dense à 18% de polyacrylamide (acrylamide 2,5 M ; Tris 0,4 M ; Glycérol 30% (v/v) ; dodécyl sulfate de sodium (SDS) 3,5 mM ; TEMED (N,N,N',N'-tétraméthyléthylènediamine ; Sigma) 0,05% (v/v) ; persulfate de sodium 1,6 mM) sont placés dans la chambre de mélange sous agitation constante tandis qu'un même volume de solution légère à 6% de polyacrylamide (acrylamide 0,8 M ; Tris 0,4 M ; SDS 3,5 mM ; TEMED 0,06% (v/v) ; persulfate de sodium 2,4 mM) est placé dans l'autre chambre. Le haut du gel est recouvert d'une solution d'isobutanol saturée en eau bidistillée. Le gel est ensuite laissé 1 h à polymériser à température ambiante puis le haut du gel est rincé plusieurs fois avec de l'eau bidistillée et l'ensemble est placé une nuit à 10°C. Après avoir enlevé l'eau résiduelle à l'aide d'un papier absorbant, la solution de gel de concentration (acrylamide 0,56 M ; méthylène bis-acrylamide 6,9 mM ; Tris 124 mM ; SDS 3,5 mM ; TEMED 0,05% (v/v) ; persulfate de sodium 2,2 mM) est coulée sur le gel de séparation et une cale permettant de former l'empreinte de la bande est introduite dans la solution gel de concentration. La polymérisation est complète après 1 h à température ambiante.

### 4) Analyse par micro séquençage des chaînes protéiques isolées par phase inverse et gel bidimensionnel

Les chaînes protéiques isolées par chromatographie en phase inverse et par gel bidimensionnel sont ensuite digérées et analysées par spectrométrie de masse LC-MS/MS sur un appareil de type ESI-Q-TOF.

### Digestion des protéines séparées par chromatographie en phase inverse.

Chaque chaîne protéique isolée est soumise à une digestion enzymatique, étape essentielle avant leur analyse par microséquençage. Les chaînes protéiques lyophilisées sont dissoutes dans une solution eau milliQ, acétonitrile contenant l'endoprotéase ; la trypsine qui hydrolyse au niveau C-terminal de la lysine et de l'arginine, donnant généralement des peptides de masses comprises entre 500 et 2500 Da, pendant au minimum 3h à température ambiante.

### Digestion des protéines séparées sur gel bidimensionnel

Chaque spot du gel est découpé pour être soumis à une digestion enzymatique. Cette étape de digestion enzymatique est essentielle. Elle consiste à hydrolyser les protéines de façon spécifique, à l'aide d'une enzyme, en plusieurs peptides.

Avant de débuter la digestion, des étapes de décoloration, de réduction et d'alkylation sont indispensables :
- les lavages successifs à l'hydrogénocarbonate d'ammonium (NH₄HCO₃) et à l'acétonitrile (ACN) permettent d'éliminer l'agent de coloration présent dans le morceau de gel,
- les réactions de réduction au dithiothréitol (DTT) et d'alkylation à l'iodoacétamide permettent l'ouverture puis le blocage des ponts disulfures formés entre deux cystéines présentes dans la séquence de la protéine et des liaisons cystéine-acrylamide.

La dernière étape de la méthode consiste à extraire du gel les peptides trypsiques à l'aide d'une solution d'extraction, composée d'acétonitrile et d'eau, additionnée d'un peu d'acide.

### Analyse par nanoLC-MS-MS

Les peptides extraits sont ensuite transférés vers la plaque PCR. Ce transfert se fait en deux fois 15 µL, pour récupérer la totalité du volume. Pour éliminer l'acétonitrile, qui pourrait gêner la rétention des peptides sur la pré-colonne, un temps d'évaporation (pause) de 2 heures est appliqué avant l'analyse par nanoLC-MS-MS.

### Résultats

Ceci a permis l'obtention de quelques centaines de micoséquences correspondant à chaque chaîne protéique séparée par gel 2D.

### 5) Analyse par séquençage d'Edman des chaînes protéiques isolées par chromatographie en phase inverse

### Principe

En présence de tampon N-méthyl pipéridine, le Phényl-Iso-Thio-Cyanate (PITC) se couple aux fonctions amines primaires et secondaires des protéines (PTC-Protéine). Le temps de réaction à 45°C est de 18 minutes. La liaison peptidique suivante est fragilisée, ce qui permet sa coupure en 3 minutes par l'acide trifluoracétique (TFA) pur générant ainsi l'anilino-thiazolinone (ATZ) du premier acide aminé (AA) et la protéine ayant perdu le 1er AA.

L'ATZ-AA est extrait du milieu réactionnel et converti en milieu acide (TFA à 25 % dans l'eau) en phényl thio-hydantoïne (PTH-AA) plus stable. Le PTH-AA peut donc être analysé par HPLC et sa nature déterminée grâce à un étalon de PTH-AAs. Le cycle de réaction peut être répété et conduit ainsi à la séquence de la protéine. Edman a automatisé la réaction qui porte son nom en créant en 1967 le premier séquenceur de protéine. L'appareil est couplé à une HPLC dans laquelle il injecte le PTH-AA. Par comparaison avec un spectre étalon, il est alors possible d'identifier l'acide aminé d'origine et d'obtenir sa quantification. Le tout est sous le contrôle d'un ordinateur qui pilote les différents éléments et assure l'acquisition de données ainsi que leur traitement.

### Résultats

Ainsi, il a été possible d'obtenir environ 30 acides aminés des extrémités N-terminales de 5 monomères et d'un linker isolés par phase inverse.

### Détails des protocoles d'amplification par PCR et présentation des séquences nucléotidiques et polypeptidiques des globines des sous-familles A1, A2a, A2b, B2 et B1 et du linker L1 du polychète marin Arenicola marina

Les amplifications par PCR des 5 globines A1, A2a, A2b, B1 et B2, ainsi que du linker L1, dont les séquences nucléotidiques sont présentées ci-dessous, ont débuté par la conception d'amorces dégénérées spécifiques (sens et antisens) des sous-familles A1, A2, B1 et B2. Ces amorces, qui ont permis l'amplification des cinq globines susmentionnées (A1, A2a, A2b, B1 et B2) puis le clonage et le séquençage des produits de PCR correspondants, ont été conçues à partir d'alignements de séquences protéiques de globines d'annélides disponibles dans les banques de données.

Les matrices d'ADN complémentaires utilisées pour les réactions de PCR ont été synthétisées à partir d'ARN messagers purifiés à partir d'ARN totaux extraits sur des Arénicoles, en raison de la petite taille des organismes et de leur rythme intense de croissance traduisant des niveaux importants d'expression des gènes, dont ceux impliqués dans la synthèse de l'hémoglobine. Les ADN complémentaires ont ainsi été synthétisés. Ces étapes ont fait appel à des kits commerciaux de biologie moléculaire de chez Ambion (purification des ARNs), Amersham (purification des ARNm), Promena (RT), Invitrogene (clonage), Abgene (séquençage).

Dans un second temps, nous avons mis au point les réactions de PCR, notamment en ce qui concerne la détermination des paramètres de temps de dénaturation, de temps et de température d'hybridation et de temps d'élongation. Les concentrations en MgCl₂ ont également été optimisées.

Enfin, dans une dernière étape, des expériences de 5' et 3' RACE PCR ont été effectuées de façon à obtenir les séquences codantes complètes. Ces étapes ont fait appel au kit de biologie moléculaire de chez Roche.

Sont présentées ci-dessous, les séquences nucléotidiques des amorces dégénérées sens et antisens, les paramètres des PCR et les séquences codantes partielles ou complètes pour chacune des globines A2a, A2b, Al, B1 et B2, et pour le linker L1.

On précise que l'analyse totale de ces séquences par un blast dans les banques de données donne des valeurs comprises entre 2.10⁻³ < Evalue < 5^{e-31}.

### Globine A2a

Pour obtenir la séquence nucléotidique SEQ ID NO : 1 codant pour la globine A2a (SEQ ID NO : 2), on utilise le couple d'amorces (SEQ ID NO : 19 ; SEQ ID NO : 20).

Les conditions de PCR sont les suivantes :

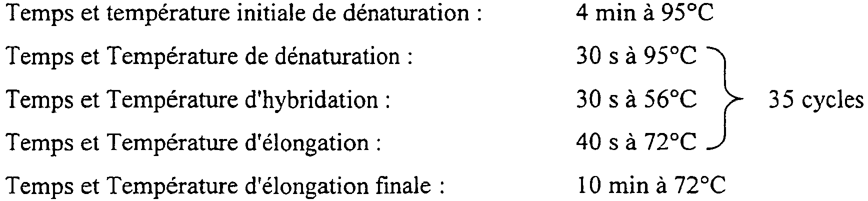

Réaction de PCR :

| | |
|---|---|
| Par réaction : | 5-20 ng ADNc |
| | 100 ng amorce sens |
| | 100 ng amorce antisens |
| | dNTP 200 µM final |
| | MgCl₂ 2mM final |
| | Tampon PCR 1X final |
| | 1 unité Taq Polymérase |
| | Qsp 25 µL H₂O |

### Globine A2b

Pour obtenir la séquence nucléotidique SEQ ID NO : 3 codant pour la globine A2b (SEQ ID NO : 4), on utilise le couple d'amorces (SEQ ID NO : 21 ; SEQ ID NO : 20).

Les conditions de PCR sont les suivantes :

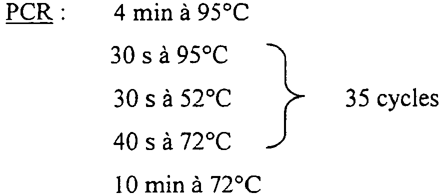

### Globine A1

Pour obtenir la séquence nucléotidique SEQ ID NO : 5 codant pour la globine A1 (SEQ ID NO : 6), on utilise le couple d'amorces (SEQ ID NO : 22 ; SEQ ID NO : 20).

Les conditions de PCR sont les suivantes :

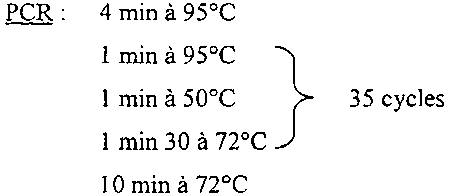

### Globine B2

Pour obtenir la séquence nucléotidique SEQ ID NO : 7 codant pour la globine B2 (SEQ ID NO : 8), on utilise le couple d'amorces (SEQ ID NO : 23 ; SEQ ID NO : 20).

Les conditions de PCR sont les suivantes :

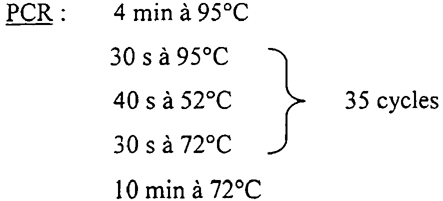

### Globine B1

Pour obtenir la séquence nucléotidique SEQ ID NO : 9 codant pour la globine B1 (SEQ ID NO : 10), on utilise le couple d'amorces (SEQ ID NO : 24 ; SEQ ID NO : 20).

Les conditions de PCR sont les suivantes :

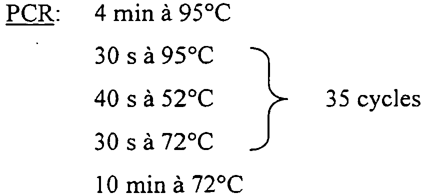

### Linker L1

Pour obtenir la séquence nucléotidique SEQ ID NO : 11 codant pour le Linker L1 (SEQ ID NO : 12), on utilise le couple d'amorces (SEQ ID NO : 25 ; SEQ ID NO : 20).

Les conditions de PCR sont les suivantes :

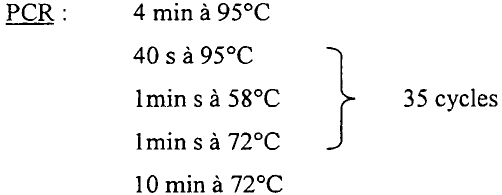

### REFERENCES BIBLIOGRAPHIQUES

- Bunn, H.F. et Jandl, J.H. (1968) Trans Assoc Am Physicians, 81, 147,
- Chang, T.M.S. (1957) Hemoglobin Corpuscles, McGill University,
- Chang, T.M.S. (1964) Science, 146, 524-525,
- Chang, T.M.S. (1971) Biochem. Biophys. Res. Com., 44, 1531-1533,
- Chang, T.M.S. (1997) Blood substitutes: principales, methods, products and clinical trials, Vol. I, Karger, Basel, Suisse,
- Chiancone, E., et al. (1972) Studies on erythrocruorin. II. Dissociation of earthworm erythrocruorin, J Mol Biol, 70(1): 73-84,
- Clark, L.C.J. et Gollan, F. (1966) Science, 152, 1755,
- De Haas, F.; Boisset, N.; Taveau, J.C.; Lambert, O.; Vinogradov, S.N. et Lamy, J.N. (1996) Biophys. J., 70, 1973-1984,
- De Haas, F.; Taveau, J.-C.; Boisset, N.; Lambert, O.; Vinogradov, S.N. et Lamy, J.N. (1996) J. Mol. Biol., 255, 140-153,
- De Haas, F.; Zal, F.; Lallier, F.H.; Toulmond, A.et Lamy, J.N. (1996) Proteins-structure fonction and genetics, 3, 241-256,
- De Haas, F.; Zal, F.; You, V.; Lallier, F.H.; Toulmond, A. et Lamy, J.N. (1996) J. Mol. Biol., 264, 111-120,
- Dickerson, R.E. et Geis, I. (1983) Hemoglobin: Structure, function, evolution, and pathology., Benjamin/Cummings, Menlo Park, CA,
- Geyer, R.P.; Monroe, R.G. et Taylor, K. (1968) Survival of rats totally perfused with a fluorocarbon-detergent preparation., J.C. Norman, J. Folkman, W.G. Hardisson, L.E. Rudolf et F.J. Veith (Eds), 85-96, Appleton Century Crofts, New York,
- Goodin, T.H.; Grossbard, E.B.; Kaufman, R.J.; Richard, T.J.; Kolata, R.J.; Allen, J.S. et Layton, T.E. (1994), Crit Care Med, 22, 680-689,
- Hirsch, R.E.; Jelicks, L.A.; Wittenberg, B.A.; Kaul, D.K.; Shear, H.L. et Harrington, J.P. (1997) Artificial Cells, Blood Substitutes & Immobilization Biotechnology, 25, 429-444,
- Jia, L.; Bonaventura, C.; Bonaventura, J. et Stamler, J.S. (1996) Nature, 380, 221-226,
- Kapp, O.H. et Crewe, A.V. (1984) Biochim. Biophys. Acta 789, 294-301,
- Kapp, O.H., et al. (1984) The reassociation of Lumbricus terrestris hemoglobin dissociated at alkaline pH, J Biol Chem, 259(1): 628-39,
- Krebs, A., et al. (1996) Molecular shape, dissociation, and oxygen binding of the dodecamer subunit of Lumbricus terrestris hemoglobin, J Biol Chem, 271(31): 18695-704,
- Lamy, J.N.; Green, B.N.; Toulmond, A., Wall, J.S.; Weber, R.E. et Vinogradov, S.N. (1996), Chem. Rev. 96, 3113-3124
- Levin, O. (1963) J. Mol. Biol., 6, 95-101,
- Mainwaring, M.G., et al. (1986) The dissociation of the extracellular hemoglobin of Lumbricus terrestris at acid pH and its reassociation at neutral pH. A new model of its quaternary structure, J Biol Chem, 261(23): 10899-908,
- Mitsuno, T. et Naito, R. (1979) Perfluorochemical Blood Substitutes., Excerpta Medica, Amsterdam,
- Mitsuno, T. et Ohyanagi, H. (1985) Present status of clinical studies of fluosol-DA (20%) in Japan, K.K. Tremper (Ed), 169-184, Little Brown & Co, Boston,
- Naito, R. et Y. K. (1978) An improved perfluorodecalin emulsion. In Blood Substitutes and Plasma Expanders, G.A. Jamieson and T.J. Greenwalt (Eds), 81, Alan R. Liss Inc., New York,
- Nho, K.; Glower, D.; Bredehoeft, S.; Shankar, H.; Shorr, R. et Abuchowski, A. (1992) Biomaterials, Artificial Cells and Immobilization Biotechnology, An International Journal, 20, 511-524,
- Payne, J.W. (1973) Biochem J. 135, 866-873,
- Polidori, G., et al. (1984) The dissociation of the extracellular hemoglobin of Tubifex tubifex at extremes of pH and its reassociation upon return to neutrality, Arch Biochem Biophys, 233(2): 800-14,
- Reiss, J.G. (1991) Vox Sang, 61, 225-239,
- Reiss, J.G., (1994) Artificial Cells, Blood substitutes & Immobilization Biotechnology, An International Journal 22, 945-1511,
- Roche, J. (1965) Electron microscope studies on high molecular weight erythrocruorins (invertebrate hemoglobins) and chlorocruorins of annelids., D.A. Munday (Ed), 62-80, Pergamon Press, Oxford,
- Roche, J.; Bessis, M. et Thiery, J.P. (1960) Biochim. Biophys. Acta, 41, 182-184,
- Roche, J.; Bessis, M.T. et Thiery, J.P. (1960) C. R. Soc. Biol. 154, 73-80,
- Sharma, P.K., et al. (1996) The role of the dodecamer subunit in the dissociation and reassembly of the hexagonal bilayer structure of Lumbricus terrestris hemoglobin, J Biol Chem, 271(15): 8754-62,
- Sloviter, H. et Kamimoto, T. (1967) Nature, 216, 458,
- Terwilliger, N.B. (1992) Molecular Structure of the extracellular heme proteins., Vol. 13, C.P. Mangum (Ed), 193-229, Springer-Verlag, Berlin,
- Van Bruggen, E.F.J. et Weber, R.E. (1974) Biochim. Biophys. Acta, 359, 210-212,
- Vinogradov, S.N., et al. (1991) A dodecamer of globin chains is the principal functional subunit of the extracellular hemoglobin of Lumbricus terrestris, J Biol Chem, 266(20): 13091-6,
- Vinogradov, S.N., Shlom J. M. et Doyle, M. (1979) Dissociation of the extracellular hemoglobin of Arenicola cristata. Comp. Biochem. Physiol., 65B: 145-150,
- Vinogradov, S.N. (1985) The structure of invertebrate extracellular hemoglobins (erythrocruorins and chlorocruorins), Comp Biochem Physiol B, 82(1): 1-15,
- Zal, F.; Green, B.N.; Lallier, F.H.; Vinogradov, S.N. et Toulmond, A. (1997) Eur. J. Biochem., 243, 85-92.

## Revendications

1. Procédé d'obtention des chaînes protéiques constituant la molécule d'hémoglobine extracellulaire d'Arenicola marina,
ledit procédé étant **caractérisé en ce qu'**il comprend soit une étape de mise en présence d'un échantillon d'hémoglobine extracellulaire d'Arenicola marina avec au moins un agent dissociant et un agent réducteur qui est un mélange constitué de dithiothréitol (DTT) ou de chlorhydrate de tris(2-carboxyéthyl)phosphine (TCEP) ou de bêta-mercaptoéthanol et d'un tampon de dissociation, pendant un temps suffisant pour séparer les chaînes protéiques les unes des autres,
soit la réduction de quatre sous-unités par un agent réducteur, lesdites sous-unités étant elles-mêmes obtenues par mise en présence d'un échantillon d'hémoglobine extracellulaire d'Arenicola marina avec un tampon de dissociation,
ledit tampon de dissociation comprenant de l'EDTA à une concentration d'environ 1 mM ajusté à un pH d'environ 10.

2. Procédé selon la revendication 1, **caractérisé en ce que** l'agent dissociant est l'urée 3M et le tampon de dissociation comprend un agent tamponnant Trisma (tris[hydroxyméthyl]aminométhane) à une concentration de 0,1 M, et 1 mM d'EDTA, ajusté à un pH de 10.

3. Couples d'amorces, lesdits couples étant les suivants :
**a) *Amorce sens :*** GAR TGY GGN CCN TTR CAR CG (SEO ID NO : 21)
***Amorce antisens :*** CTC CTC TCC TCT CCT CTT CCT (SEQ ID NO : 22)
***b) Amorce sens :*** TGY GGN ATH CTN CAR CG (SEQ ID NO : 23)
***Amorce antisens :*** CTC CTC TCC TCT CCT CTT CCT (SEQ ID NO : 22)
c) ***Amorce sens :*** AAR GTI AAR CAN AAC TGG (SEQ ID NO : 24)
***Amorce antisens :*** CTC CTC TCC TCT CCT CTT CCT (SEQ ID NO : 22)
d) ***Amorce sens :*** TGY TGY AGY ATH GAR GAY CG (SEQ ID NO : 25)
***Amorce antisens :*** CTC CTC TCC TCT CCT CTT CCT (SEQ ID NO : 22)
**e) *Amorce sens :*** AAR GTN ATH TTY GGN AGR GA (SEO ID NO : 26)
***Amorce antisens:*** CTC CTC TCC TCT CCT CTT CCT (SEQ ID NO : 22)
**f) *Amorce sens :*** GAR CAY CAR TGY GGN GGN GA (SEQ ID NO : 27)
***Amorce antisens :*** CTC CTC TCC TCT CCT CTT CCT (SEO ID NO : 22)
où :
R représente A ou G,
Y représente C ou T,
N représente A, Gy C ou T,
I représente l'inosine,
H représente A, C ou T.

4. Protéines codées par l'une des séquences SEQ ID Nos : 1, 3, 5, 7, 9, 11, 13, 15, 17, 19.

5. Protéine selon la revendication 4, **caractérisée en ce qu'**elle comprend ou est constituée par :
- la séquence SEQ ID NO : 2,
- ou tout fragment de la séquence définie ci-dessus, sous réserve que ledit fragment permette le transport de l'oxygène, ledit fragment étant constitué d'au moins 60 acides aminés contigus dans la séquence SEQ ID NO : 2.

6. Protéine selon la revendication 4, **caractérisée en ce qu'**elle comprend ou est constituée par :
- la séquence SEQ ID NO : 4,
- ou tout fragment de la séquence définie ci-dessus, sous réserve que ledit fragment permette le transport de l'oxygène, ledit fragment étant constitué d'au moins environ 60 acides aminés contigus dans la séquence SEQ ID NO : 4.

7. Protéine selon la revendication 4, **caractérisée en ce qu'**elle comprend ou est constituée par :
- la séquence SEQ ID NO : 6,
- ou tout fragment de la séquence définie ci-dessus, sous réserve que ledit fragment permette le transport de l'oxygène, ledit fragment étant constitué d'au moins environ 60 acides aminés contigus dans la séquence SEQ ID NO : 6.

8. Protéine selon la revendication 4, **caractérisée en ce qu'**elle comprend ou est constituée par :
- la séquence SEQ ID NO : 8,
- ou tout fragment de la séquence définie ci-dessus, sous réserve que ledit fragment permette le transport de l'oxygène, ledit fragment étant constitué d'au moins environ 60 acides aminés contigus dans la séquence SEQ ID NO : 8.

9. Protéine selon la revendication 4, **caractérisée en ce qu'**elle comprend ou est constituée par :
- la séquence SEQ ID NO : 10,
- ou tout fragment de la séquence définie ci-dessus, sous réserve que ledit fragment permette le transport de l'oxygène, ledit fragment étant constitué d'au moins environ 60 acides aminés contigus dans la séquence SEQ ID NO : 10.

10. Protéine selon la revendication 4, **caractérisée en ce qu'**elle comprend ou est constituée par :
- la séquence SEQ ID NO : 12,
- ou tout fragment de la séquence définie ci-dessus, sous réserve que ledit fragment permette l'association des chaînes de globines entre elles, ledit fragment étant constitué d'au moins environ 60 acides aminés contigus dans la séquence SEQ ID NO : 12.

11. Séquences nucléotidiques SEQ ID Nos : 1, 3, 5, 7, 9, 11, 13, 15, 17, 19.

12. Séquences nucléotidiques codant pour une protéine telle que définie dans l'une des revendications 4 à 10.

13. Séquence nucléotidique selon la revendication 12, **caractérisée en ce qu'**elle comprend ou est constituée par :
- la séquence nucléotidique SEQ ID NO : 1 codant pour SEQ ID NO : 2,
- ou toute séquence nucléotidique dérivée, par dégénérescence du code génétique, de la séquence SEQ ID NO : 1, et codant pour une protéine représentée par SEQ ID NO : 2,
- ou tout fragment de la séquence nucléotidique SEQ ID NO : 1 ou des séquences nucléotidiques définies ci-dessus, ledit fragment étant constitué d'au moins environ 180 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

14. Séquence nucléotidique selon la revendication 12, **caractérisée en ce qu'**elle comprend ou est constituée par :
- la séquence nucléotidique SEQ ID NO : 3 codant pour SEQ ID NO : 4,
- ou toute séquence nucléotidique dérivée, par dégénérescence du code génétique, de la séquence SEQ ID NO : 3, et codant pour une protéine représentée par SEQ ID NO : 4,
- ou tout fragment de la séquence nucléotidique SEQ ID NO : 3 ou des séquences nucléotidiques définies ci-dessus, ledit fragment étant constitué d'au moins environ 180 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

15. Séquence nucléotidique selon la revendication 12, **caractérisée en ce qu'**elle comprend ou est constituée par :
- la séquence nucléotidique SEQ ID NO : 5 codant pour SEQ ID NO : 6,
- ou toute séquence nucléotidique dérivée, par dégénérescence du code génétique, de la séquence SEQ ID NO : 5, et codant pour une protéine représentée par SEQ ID NO : 6,
- ou tout fragment de la séquence nucléotidique SEQ ID NO : 5 ou des séquences nucléotidiques définies ci-dessus, ledit fragment étant constitué d'au moins environ 180 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

16. Séquence nucléotidique selon la revendication 12, **caractérisée en ce qu'**elle comprend ou est constituée par :
- la séquence nucléotidique SEQ ID NO : 7 codant pour SEQ ID NO : 8,
- ou toute séquence nucléotidique dérivée, par dégénérescence du code génétique, de la séquence SEQ ID NO : 7, et codant pour une protéine représentée par SEQ ID NO : 8,
- ou tout fragment de la séquence nucléotidique SEQ ID NO : 7 ou des séquences nucléotidiques définies ci-dessus, ledit fragment étant constitué d'au moins environ 180 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

17. Séquence nucléotidique selon la revendication 12, **caractérisée en ce qu'**elle comprend ou est constituée par :
- la séquence nucléotidique SEQ ID NO : 9 codant pour SEQ ID NO : 10,
- ou toute séquence nucléotidique dérivée, par dégénérescence du code génétique, de la séquence SEQ ID NO : 9, et codant pour une protéine représentée par SEQ ID NO : 10,
- ou tout fragment de la séquence nucléotidique SEQ ID NO : 9 ou des séquences nucléotidiques définies ci-dessus, ledit fragment étant constitué d'au moins environ 180 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

18. Séquence nucléotidique selon la revendication 12, **caractérisée en ce qu'**elle comprend ou est constituée par :
- la séquence nucléotidique SEQ ID NO : 11 codant pour SEQ ID NO : 12,
- ou tout fragment de la séquence nucléotidique SEQ ID NO : 11 ou des séquences nucléotidiques définies ci-dessus, ledit fragment étant constitué d'au moins environ 180 nucléotides contigus dans ladite séquence,
- ou toute séquence nucléotidique complémentaire des séquences ou fragments susmentionnés,
- ou toute séquence nucléotidique susceptible d'hybrider dans des conditions stringentes avec la séquence complémentaire d'une des séquences ou d'un des fragments susmentionnés.

## Claims

1. Method for obtaining protein chains constituting the extracellular haemoglobin molecule of Arenicola marina,
said method being **characterized in that** it comprises either a stage of bringing together a sample of extracellular haemoglobin of Arenicola marina and at least one dissociating agent and a reducing agent, which is a mixture made up of dithiothreitol (DTT) or tris(2-carboxyethyl)phosphine hydrochloride (TCEP) or beta-mercaptoethanol and a dissociation buffer, for a sufficient time to separate the protein chains from each other,
or the reduction of four sub-units by a reducing agent, said sub-units themselves being obtained by bringing together a sample of extracellular haemoglobin of Arenicola marina and a dissociation buffer,
said dissociation buffer comprising EDTA at a concentration of around 1 mM adjusted to a pH of around 10.

2. Method according to claim 1, **characterized in that** the dissociating agent is urea 3M and the dissociation buffer comprises a buffering agent Trisma (tris[hydroxymethyl]aminomethane) at a concentration of 0.1 M, and 1 mM of EDTA adjusted to a pH of 10.

3. Primer pairs, said pairs being the following:
a) Sense primer: GAR TGY GGN CCN TTR CAR CG (SEQ ID NO: 21)
Antisense primer: CTC CTC TCC TCT CCT CTT CCT (SEQ ID NO: 22)
b) Sense primer: TGY GGN ATH CTN CAR CG (SEQ ID NO: 23)
Antisense primer: CTC CTC TCC TCT CCT CTT CCT (SEQ ID NO: 22)
c) Sense primer: AAR GTI AAR CAN AAC TGG (SEQ ID NO: 24)
Antisense primer: CTC CTC TCC TCT CCT CTT CCT (SEQ ID NO: 22)
d) Sense primer: TGY TGY AGY ATH GAR GAY CG (SEQ ID NO: 25)
Antisense primer: CTC CTC TCC TCT CCT CTT CCT (SEQ ID NO: 22)
e) Sense primer: AAR GTN ATH TTY GGN AGR GA (SEQ ID NO: 26)
Antisense primer: CTC CTC TCC TCT CCT CTT CCT (SEQ ID NO: 22)
f) Sense primer: GAR CAY CAR TGY GGN GGN GA (SEQ ID NO: 27)
Antisense primer: CTC CTC TCC TCT CCT CTT CCT (SEQ ID NO: 22)
where:
R represents A or G,
Y represents C or T,
N represents A, G, C or T,
I represents inosine,
H represents A, C or T.

4. Proteins encoded by one of the sequences SEQ ID NO: 1, 3, 5, 7, 9, 11, 13, 15, 17, 19.

5. Protein according to claim 4, **characterized in that** it comprises or is constituted by:
- the sequence SEQ ID NO: 2,
- or any fragment of the sequence defined above, provided that said fragment allows the transport of oxygen, said fragment being made up of at least 60 contiguous amino acids in the sequence SEQ ID NO: 2.

6. Protein according to claim 4, **characterized in that** it comprises or is constituted by:
- the sequence SEQ ID NO: 4,
- or any fragment of the sequence defined above, provided that said fragment allows the transport of oxygen, said fragment being made up of at least around 60 contiguous amino acids in the sequence SEQ ID NO: 4.

7. Protein according to claim 4, **characterized in that** it comprises or is constituted by:
- the sequence SEQ ID NO: 6,
- or any fragment of the sequence defined above, provided that said fragment allows the transport of oxygen, said fragment being made up of at least around 60 contiguous amino acids in the sequence SEQ ID NO: 6.

8. Protein according to claim 4, **characterized in that** it comprises or is constituted by:
- the sequence SEQ ID NO: 8,
- or any fragment of the sequence defined above, provided that said fragment allows the transport of oxygen, said fragment being made up of at least around 60 contiguous amino acids in the sequence SEQ ID NO: 8.

9. Protein according to claim 4, **characterized in that** it comprises or is constituted by:
- the sequence SEQ ID NO: 10,
- or any fragment of the sequence defined above, provided that said fragment allows the transport of oxygen, said fragment being made up of at least around 60 contiguous amino acids in the sequence SEQ ID NO: 10.

10. Protein according to claim 4, **characterized in that** it comprises or is constituted by:
- the sequence SEQ ID NO: 12,
- or any fragment of the sequence defined above, provided that said fragment allows the combination of globin chains with each other, said fragment being made up of at least around 60 contiguous amino acids in the sequence SEQ ID NO: 12.

11. Nucleotide sequences SEQ ID NO: 1, 3, 5, 7, 9, 11, 13, 15, 17, 19.

12. Nucleotide sequences encoding a protein as defined in any one of claims 4 to 10.

13. Nucleotide sequence according to claim 12, **characterized in that** it comprises or is constituted by:
- the nucleotide sequence SEQ ID NO: 1 encoding SEQ ID NO: 2,
- or any nucleotide sequence derived, by degeneration of the genetic code, from the sequence SEQ ID NO: 1, and encoding a protein represented by SEQ ID NO: 2,
- or any fragment of the nucleotide sequence SEQ ID NO: 1 or of the nucleotide sequences defined above, said fragment being constituted by at least approximately 180 contiguous nucleotides in said sequence,
- or any nucleotide sequence complementary to the abovementioned sequences or fragments,
- or any nucleotide sequence capable of hybridizing under stringent conditions with the sequence complementary to one of the abovementioned sequences or fragments.

14. Nucleotide sequence according to claim 12, **characterized in that** it comprises or is constituted by:
- the nucleotide sequence SEQ ID NO: 3 encoding SEQ ID NO: 4,
- or any nucleotide sequence derived, by degeneration of the genetic code, from the sequence SEQ ID NO: 3, and encoding a protein represented by SEQ ID NO: 4,
- or any fragment of the nucleotide sequence SEQ ID NO: 3 or of the nucleotide sequences defined above, said fragment being constituted by at least approximately 180 contiguous nucleotides in said sequence,
- or any nucleotide sequence complementary to the abovementioned sequences or fragments,
- or any nucleotide sequence capable of hybridizing under stringent conditions with the sequence complementary to one of the abovementioned sequences or fragments.

15. Nucleotide sequence according to claim 12, **characterized in that** it comprises or is constituted by:
- the nucleotide sequence SEQ ID NO: 5 encoding SEQ ID NO: 6,
- or any nucleotide sequence derived, by degeneration of the genetic code, from the sequence SEQ ID NO: 5, and encoding a protein represented by SEQ ID NO: 6,
- or any fragment of the nucleotide sequence SEQ ID NO: 5 or of the nucleotide sequences defined above, said fragment being constituted by at least approximately 180 contiguous nucleotides in said sequence,
- or any nucleotide sequence complementary to the abovementioned sequences or fragments,
- or any nucleotide sequence capable of hybridizing under stringent conditions with the sequence complementary to one of the abovementioned sequences or fragments.

16. Nucleotide sequence according to claim 12, **characterized in that** it comprises or is constituted by:
- the nucleotide sequence SEQ ID NO: 7 encoding SEQ ID NO: 8,
- or any nucleotide sequence derived, by degeneration of the genetic code, from the sequence SEQ ID NO: 7, and encoding a protein represented by SEQ ID NO: 8,
- or any fragment of the nucleotide sequence SEQ ID NO: 7 or of the nucleotide sequences defined above, said fragment being constituted by at least approximately 180 contiguous nucleotides in said sequence,
- or any nucleotide sequence complementary to the abovementioned sequences or fragments,
- or any nucleotide sequence capable of hybridizing under stringent conditions with the sequence complementary to one of the abovementioned sequences or fragments.

17. Nucleotide sequence according to claim 12, **characterized in that** it comprises or is constituted by:
- the nucleotide sequence SEQ ID NO: 9 encoding SEQ ID NO: 10,
- or any nucleotide sequence derived, by degeneration of the genetic code, from the sequence SEQ ID NO: 9, and encoding a protein represented by SEQ ID NO: 10,
- or any fragment of the nucleotide sequence SEQ ID NO: 9 or of the nucleotide sequences defined above, said fragment being constituted by at least approximately 180 contiguous nucleotides in said sequence,
- or any nucleotide sequence complementary to the abovementioned sequences or fragments,
- or any nucleotide sequence capable of hybridizing under stringent conditions with the sequence complementary to one of the abovementioned sequences or fragments.

18. Nucleotide sequence according to claim 12, **characterized in that** it comprises or is constituted by:
- the nucleotide sequence SEQ ID NO: 11 encoding SEQ ID NO: 12,
- or any fragment of the nucleotide sequence SEQ ID NO: 11 or of the nucleotide sequences defined above, said fragment being constituted by at least approximately 180 contiguous nucleotides in said sequence,
- or any nucleotide sequence complementary to the abovementioned sequences or fragments,
- or any nucleotide sequence capable of hybridizing under stringent conditions with the sequence complementary to one of the abovementioned sequences or fragments.

## Patentansprüche

1. Verfahren zur Gewinnung von Proteinketten, die das extrazelluläre Hämoglobinmolekül von Arenicola marina bilden,
wobei das Verfahren **dadurch gekennzeichnet ist, dass** es entweder einen Schritt des Zusammenbringens einer Probe des extrazellulären Hämoglobins von Arenicola marina mit mindestens einem Dissoziationsmittel und einem Reduktionsmittel, das eine Mischung bestehend aus Dithiothreitol (DTT) oder Tris-(2-carboxyethyl)-phosphin hydrochlorid (TCEP) oder beta-Mercaptoethanol und einem Dissoziationspuffer ist, für eine Zeit, die ausreichend ist, um die Proteinketten voneinander zu trennen, umfasst,
oder die Reduktion von vier Untereinheiten durch ein Reduktionsmittel, wobei die Untereinheiten selbst durch Zusammenbringen einer Probe des extrazellulären Hämoglobins von Arenicola marina mit einem Dissoziationspuffer erhalten werden,
wobei der Dissoziationspuffer EDTA in einer Konzentration von ungefähr 1 mM umfasst, eingestellt auf einen pH-Wert von ungefähr 10.

2. Verfahren gemäß Anspruch 1, **dadurch gekennzeichnet, dass** das Dissoziationsmittel 3 M Harnstoff ist und der Dissoziationspuffer ein Pufferungsmittel Trisma (Tris(hydroxymethyl)-aminomethan) in einer Konzentration von 0,1 M und 1 mM EDTA enthält, eingestellt auf einen pH-Wert von 10.

3. Primerpaare, wobei die Paare die folgenden sind:
a) Sense-Primer: GAR TGY GGN CCN TTR CAR CG (SEQ ID NO: 21)
Antisense-Primer: CTC CTC TCC TCT CCT CTT CCT (SEQ ID NO: 22)
b) Sense-Primer: TGY GGN ATH CTN CAR CG (SEQ ID NO: 23)
Antisense-Primer: CTC CTC TCC TCT CCT CTT CCT (SEQ ID NO: 22)
c) Sense-Primer: AAR GTI AAR CAN AAC TGG (SEQ ID NO: 24)
Antisense-Primer: CTC CTC TCC TCT CCT CTT CCT (SEQ ID NO: 22)
d) Sense-Primer: TGY TGY AGY ATH GAR GAY CG (SEQ ID NO: 25)
Antisense-Primer: CTC CTC TCC TCT CCT CTT CCT (SEQ ID NO: 22)
e) Sense-Primer: AAR GTN ATH TTY GGN AGR GA (SEQ ID NO: 26)
Antisense-Primer: CTC CTC TCC TCT CCT CTT CCT (SEQ ID NO: 22)
f) Sense-Primer: GAR CAY CAR TGY GGN GGN GA (SEQ ID NO: 27)
Antisense-Primer: CTC CTC TCC TCT CCT CTT CCT (SEQ ID NO: 22)
wobei:
R A oder G darstellt,
Y C oder T darstellt,
N A, G, C oder T darstellt,
I Inosin darstellt,
H A, C oder T darstellt.

4. Proteine, kodiert durch eine der Sequenzen der SEQ ID NO: 1, 3, 5, 7, 9, 11, 13, 15, 17, 19.

5. Protein gemäß Anspruch 4, **dadurch gekennzeichnet, dass** es umfasst oder gebildet wird durch:
- die Sequenz der SEQ ID NO: 2,
- oder irgendein Fragment der oben definierten Sequenz, unter dem Vorbehalt, dass das Fragment den Transport von Sauerstoff erlaubt, wobei das Fragment aus mindestens 60 aufeinanderfolgenden Aminosäuren in der Sequenz der SEQ ID NO: 2 gebildet ist.

6. Protein gemäß Anspruch 4, **dadurch gekennzeichnet, dass** es umfasst oder gebildet wird durch:
- die Sequenz der SEQ ID NO: 4,
- oder irgendein Fragment der oben definierten Sequenz, unter dem Vorbehalt, dass das Fragment den Transport von Sauerstoff erlaubt, wobei das Fragment aus mindestens 60 aufeinanderfolgenden Aminosäuren in der Sequenz der SEQ ID NO: 4 gebildet ist.

7. Protein gemäß Anspruch 4, **dadurch gekennzeichnet, dass** es umfasst oder gebildet wird durch:
- die Sequenz der SEQ ID NO: 6,
- oder irgendein Fragment der oben definierten Sequenz, unter dem Vorbehalt, dass das Fragment den Transport von Sauerstoff erlaubt, wobei das Fragment aus mindestens 60 aufeinanderfolgenden Aminosäuren in der Sequenz der SEQ ID NO: 6 gebildet ist.

8. Protein gemäß Anspruch 4, **dadurch gekennzeichnet, dass** es umfasst oder gebildet wird durch:
- die Sequenz der SEQ ID NO: 8,
- oder irgendein Fragment der oben definierten Sequenz, unter dem Vorbehalt, dass das Fragment den Transport von Sauerstoff erlaubt, wobei das Fragment aus mindestens 60 aufeinanderfolgenden Aminosäuren in der Sequenz der SEQ ID NO: 8 gebildet ist.

9. Protein gemäß Anspruch 4, **dadurch gekennzeichnet, dass** es umfasst oder gebildet wird durch:
- die Sequenz der SEQ ID NO: 10,
- oder irgendein Fragment der oben definierten Sequenz, unter dem Vorbehalt, dass das Fragment den Transport von Sauerstoff erlaubt, wobei das Fragment aus mindestens 60 aufeinanderfolgenden Aminosäuren in der Sequenz der SEQ ID NO: 10 gebildet ist.

10. Protein gemäß Anspruch 4, **dadurch gekennzeichnet, dass** es umfasst oder gebildet wird durch:
- die Sequenz der SEQ ID NO: 12,
- oder irgendein Fragment der oben definierten Sequenz, unter dem Vorbehalt, dass das Fragment den Transport von Sauerstoff erlaubt, wobei das Fragment aus mindestens 60 aufeinanderfolgenden Aminosäuren in der Sequenz der SEQ ID NO: 12 gebildet ist.

11. Nukleotidsequenzen der SEQ ID NO: 1, 3, 5, 7, 9, 11, 13, 15, 17, 19.

12. Nukleotidsequenzen, die ein Protein, wie in einem der Ansprüche 4 bis 10 definiert, kodieren.

13. Nukleotidsequenz gemäß Anspruch 12, **dadurch gekennzeichnet, dass** sie umfasst oder gebildet wird durch:
- die Nukleotidsequenz der SEQ ID NO: 1, die die SEQ ID NO: 2 kodiert,
- oder irgendeine Nukleotidsequenz, die durch die Degeneration des genetischen Codes von der Sequenz der SEQ ID NO: 1 abgeleitet ist und die ein Protein, dargestellt durch die SEQ ID NO: 2, kodiert,
- oder irgendein Fragment der Nukleotidsequenz der SEQ ID NO: 1 oder der oben definierten Nukleotidsequenzen, wobei das Fragment aus mindestens ungefähr 180 aufeinanderfolgenden Nukleotiden in dieser Sequenz gebildet ist,
- oder irgendeine Nukleotidsequenz, die zu den oben erwähnten Sequenzen oder Fragmenten komplementär ist,
- oder irgendeine Nukleotidsequenz, die unter stringenten Bedingungen mit der komplementären Sequenz einer der oben erwähnten Sequenzen oder eines der oben erwähnten Fragmente hybridisieren kann.

14. Nukleotidsequenz gemäß Anspruch 12, **dadurch gekennzeichnet, dass** sie umfasst oder gebildet wird durch:
- die Nukleotidsequenz der SEQ ID NO: 3, die die SEQ ID NO: 4 kodiert,
- oder irgendeine Nukleotidsequenz, die durch die Degeneration des genetischen Codes von der Sequenz der SEQ ID NO: 3 abgeleitet ist und die ein Protein, dargestellt durch die SEQ ID NO: 4, kodiert,
- oder irgendein Fragment der Nukleotidsequenz der SEQ ID NO: 3 oder der oben definierten Nukleotidsequenzen, wobei das Fragment aus mindestens ungefähr 180 aufeinanderfolgenden Nukleotiden in dieser Sequenz gebildet ist,
- oder irgendeine Nukleotidsequenz, die zu den oben erwähnten Sequenzen oder Fragmenten komplementär ist,
- oder irgendeine Nukleotidsequenz, die unter stringenten Bedingungen mit der komplementären Sequenz einer der oben erwähnten Sequenzen oder eines der oben erwähnten Fragmente hybridisieren kann.

15. Nukleotidsequenz gemäß Anspruch 12, **dadurch gekennzeichnet, dass** sie umfasst oder gebildet wird durch:
- die Nukleotidsequenz der SEQ ID NO: 5, die die SEQ ID NO: 6 kodiert,
- oder irgendeine Nukleotidsequenz, die durch die Degeneration des genetischen Codes von der Sequenz der SEQ ID NO: 5 abgeleitet ist und die ein Protein, dargestellt durch die SEQ ID NO: 6, kodiert,
- oder irgendein Fragment der Nukleotidsequenz der SEQ ID NO: 5 oder der oben definierten Nukleotidsequenzen, wobei das Fragment aus mindestens ungefähr 180 aufeinanderfolgenden Nukleotiden in dieser Sequenz gebildet ist,
- oder irgendeine Nukleotidsequenz, die zu den oben erwähnten Sequenzen oder Fragmenten komplementär ist,
- oder irgendeine Nukleotidsequenz, die unter stringenten Bedingungen mit der komplementären Sequenz einer der oben erwähnten Sequenzen oder eines der oben erwähnten Fragmente hybridisieren kann.

16. Nukleotidsequenz gemäß Anspruch 12, **dadurch gekennzeichnet, dass** sie umfasst oder gebildet wird durch:
- die Nukleotidsequenz der SEQ ID NO: 7, die die SEQ ID NO: 8 kodiert,
- oder irgendeine Nukleotidsequenz, die durch die Degeneration des genetischen Codes von der Sequenz der SEQ ID NO: 7 abgeleitet ist und die ein Protein, dargestellt durch die SEQ ID NO: 8, kodiert,
- oder irgendein Fragment der Nukleotidsequenz der SEQ ID NO: 7 oder der oben definierten Nukleotidsequenzen, wobei das Fragment aus mindestens ungefähr 180 aufeinanderfolgenden Nukleotiden in dieser Sequenz gebildet ist,
- oder irgendeine Nukleotidsequenz, die zu den oben erwähnten Sequenzen oder Fragmenten komplementär ist,
- oder irgendeine Nukleotidsequenz, die unter stringenten Bedingungen mit der komplementären Sequenz einer der oben erwähnten Sequenzen oder eines der oben erwähnten Fragmente hybridisieren kann.

17. Nukleotidsequenz gemäß Anspruch 12, **dadurch gekennzeichnet, dass** sie umfasst oder gebildet wird durch:
- die Nukleotidsequenz der SEQ ID NO: 9, die die SEQ ID NO: 10 kodiert,
- oder irgendeine Nukleotidsequenz, die durch die Degeneration des genetischen Codes von der Sequenz der SEQ ID NO: 9 abgeleitet ist und die ein Protein, dargestellt durch die SEQ ID NO: 10, kodiert,
- oder irgendein Fragment der Nukleotidsequenz der SEQ ID NO: 9 oder der oben definierten Nukleotidsequenzen, wobei das Fragment aus mindestens ungefähr 180 aufeinanderfolgenden Nukleotiden in dieser Sequenz gebildet ist,
- oder irgendeine Nukleotidsequenz, die zu den oben erwähnten Sequenzen oder Fragmenten komplementär ist,
- oder irgendeine Nukleotidsequenz, die unter stringenten Bedingungen mit der komplementären Sequenz einer der oben erwähnten Sequenzen oder eines der oben erwähnten Fragmente hybridisieren kann.

18. Nukleotidsequenz gemäß Anspruch 12, **dadurch gekennzeichnet, dass** sie umfasst oder gebildet wird durch:
- die Nukleotidsequenz der SEQ ID NO: 11, die die SEQ ID NO: 12 kodiert,
- oder irgendein Fragment der Nukleotidsequenz der SEQ ID NO: 11 oder der oben definierten Nukleotidsequenzen, wobei das Fragment aus mindestens ungefähr 180 aufeinanderfolgenden Nukleotiden dieser Sequenz gebildet ist,
- oder irgendeine Nukleotidsequenz, die zu den oben erwähnten Sequenzen oder Fragmenten komplementär ist,
- oder irgendeine Nukleotidsequenz, die unter stringenten Bedingungen mit der komplementären Sequenz einer der oben erwähnten Sequenzen oder eines der oben erwähnten Fragmente hybridisieren kann.
